(19) Europäisches Patentamt
European Patent Office
Office européen des brevets

(11) Veröffentlichungsnummer: **0 135 472**
**B1**

(12) # EUROPÄISCHE PATENTSCHRIFT

(45) Veröffentlichungstag der Patentschrift: **18.01.89**

(51) Int. Cl.⁴: **C 07 D 239/42**, C 07 D 239/46, C 07 D 239/52, C 07 D 239/56, C 07 D 401/12, A 01 N 43/54

(21) Anmeldenummer: **84810353.7**

(22) Anmeldetag: **19.07.84**

(54) **N-(2-Nitrophenyl)-2-aminopyrimidin-Derivate, deren Herstellung und Verwendung.**

(30) Priorität: **25.07.83 CH 4049/83**

(43) Veröffentlichungstag der Anmeldung: **27.03.85 Patentblatt 85/13**

(45) Bekanntmachung des Hinweises auf die Patenterteilung: **18.01.89 Patentblatt 89/3**

(84) Benannte Vertragsstaaten: **AT BE CH DE FR GB IT LI LU NL SE**

(56) Entgegenhaltungen: **EP-A- 0 013 143**
**FR-M- 7 324**

(73) Patentinhaber: **CIBA-GEIGY AG, Klybeckstrasse 141, CH-4002 Basel (CH)**

(72) Erfinder: **Hubele, Adolf, Dr., Obere Egg 9, CH-4312 Magden (CH)**
Erfinder: **Zondler, Helmut, Dr., Oberwilerstrasse 49, CH-4103 Bottmingen (CH)**
Erfinder: **Riebli, Peter, Bünten 17, CH-4446 Buckten (CH)**
Erfinder: **Eckhardt, Wolfgang, Dr., Breslaustrasse 14, D-7850 Lörrach (DE)**

**EP 0 135 472 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N(2-Nitrophenyl)-2-amino-pyrimidin-Derivate der nachstehenden Formel I.

Die Erfindung betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen und Bakterien.

Bei den erfindungsgemässen Verbindungen handelt es sich um solche der allgemeinen Formel I:

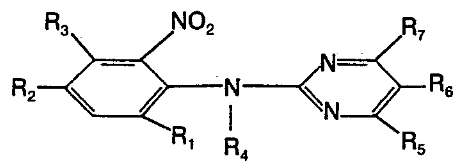 (I)

worin

$R_1$ und $R_2$ für Wasserstoff, $NO_2$ oder $CF_3$ stehen, mit der Massgabe, dass nur $R_1$ oder $R_2$ für $NO_2$ stehen kann;

$R_3$ Halogen bedeutet;

$R_4$ für Wasserstoff oder die Gruppe $-C(O)R'$ steht, wobei R' unsubstituiertes oder durch Halogen, $C_1$–$C_3$-Alkoxy oder $C_1$–$C_3$-Alkylthio substituiertes $C_1$–$C_4$-Alkyl bedeutet;

$R_5$, $R_6$ und $R_7$ unabhängig voneinander für Wasserstoff, Halogen, Nitro, Cyano, Rhodano, $C_1$–$C_{12}$-Alkyl, $C_3$–$C_8$-Cycloalkyl, $C_1$–$C_8$-Alkylthio, $C_1$–$C_6$-Alkylsulfonyl, $C_1$–$C_6$-Alkylsulfoxyl, $C_3$–$C_6$-Alkenyl, $C_3$–$C_6$-Haloalkenyl, $C_3$–$C_6$-Alkinyl, $C_3$–$C_6$-Haloalkinyl, $C_3$–$C_6$-Alkenyloxy, $C_3$–$C_6$-Haloalkenyloxy, $C_3$–$C_6$-Alkenylthio, $C_3$–$C_6$-Alkinyloxy, $C_3$–$C_6$-Haloalkinyloxy, durch Halogen, Cyano und/oder $C_1$–$C_4$-Alkoxy substituiertes $C_1$–$C_8$-Alkyl, unsubstituiertes oder durch Halogen substituiertes $C_1$–$C_8$-Alkoxy, dessen Alkylteil gegebenenfalls durch ein oder mehrere einzelne Sauerstoffatome unterbrochen ist oder für die Gruppe $Q-(E)_n-(X)_m-$ stehen, wobei n für 0 oder 1 steht, m für 0 oder 1 steht, Q für ein unsubstituiertes oder durch Halogen, Nitro, $C_1$–$C_3$-Alkyl, $CF_3$ und/oder $C_1$–$C_3$-Alkoxy substituiertes Phenyl oder einen gesättigten oder ungesättigten heterocyclischen Rest mit einem oder mehreren Heteroatomen steht, E eine $C_1$–$C_3$-Alkylenbrücke bedeutet und X für Sauerstoff oder Schwefel steht.

Unter dem Begriff Alkyl selbst oder als Bestandteil eines anderen Substituenten, wie Alkoxy, Haloalkyl, Haloalkoxy etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl, Heptyl, Octyl, Nonyl, Decyl, Undecyl, Dodecyl usw. sowie ihre Isomeren, wie z.B. Isopropyl, Isobutyl, tert.-Butyl, Isopentyl usw. Die Vorsilbe Halo in der Bezeichnung eines Substituenten bedeutet hier und im folgenden, dass dieser Substituent einfach bis perhalogeniert auftreten kann. Halogen und Halo stehen stellvertretend für Fluor, Chlor, Brom oder Jod. Haloalkyl steht somit für einen einfach bis perhalogenierten Alkylrest, wie z.B. $CHCl_2$, $CH_2F$, $CCl_3$ $CH_2Cl$, $CHF_2$, $CH_2CH_2Br$, $C_2Cl_5$, CHBr, CHBrCl usw., vorzugsweise für $CF_3$. Alkenyl steht z.B. für Propenyl-(1), Allyl, Butenyl-(1), Butenyl-(2) oder Butenyl-(3) sowie Ketten mit mehreren Doppelbindungen. Cycloalkyl steht je nach Zahl der genannten Kohlenstoffatome z.B. für Cyclopropyl, Cyclobutyl, Cyclopentyl, Cyclohexyl, Cycloheptyl, Cyclooctyl usw. Alkinyl bedeutet z.B. Propinyl-(2), Propargyl, Butinyl-(1), Butinyl-(2) usw., vorzugsweise Propargyl.

Unter einem gesättigten oder ungesättigten heterocyclischen Rest mit einem oder mehreren Heteroatomen soll hier und im folgenden vorzugsweise ein gesättigter oder ungesättigter fünf- oder sechsgliedriger Heterocyclus verstanden werden mit ein bis drei gleichen oder verschiedenen Heteroatomen, wie z.B. Sauerstoff, Stickstoff oder Schwefel. Typische Vertreter derartiger Heterocyclen sind:
Tetrahydrofuran, Furan, Tetrahydrothiophen, Thiophen, Pyrrolidin, Pyrrol, Pyrrolin, Pyrazol, Imidazol, Pyrazolin, Oxazol, Thiazol, Isoxazol, Isothiazol, Pyran, Dihydropyran, Tetrahydropyran, Thiopyran, Dihydrothiopyran, Tetrahydrothiopyran, Pyridazin, Dihydropyridazin, Tetrahydropyridazin, Pyrimidin, Dihydropyrimidin, Tetrahydropyrimidin, Pyrazin, Dihydropyrazin, Tetrahydropyrazin, Morpholin, Thiazin, Dihydrothiazin, Tetrahydrothiazin, Piperazin und Triazin. $C_1$–$C_3$-Alkylen steht z.B. für folgende Gruppen: $-CH_2-$, $-CH_2CH_2-$, $-CH_2CH_2CH_2-$, $-CH(CH_3)-CH_2-$, $-CH_2CH(CH_3)-$, $-CH(C_2H_5)-$, $-C(CH_3)_2-$.

Verbindungen vom Phenylaminopyrimidin-Typ sind bereits verschiedentlich bekannt. So sind derartige Derivate beispielsweise beschrieben in der europäischen Patentanmeldung EP 013 143 als Herbizide, in der französischen Patentschrift FR-1 324 mit pharmakologischer, insbesondere antiphlogistischer Wirkung, und in J. Heterocyclic Chem. 10(2) 167–171 (1973) ohne Angabe biologischer Eigenschaften.

Die Verbindungen der Formel I sind bei Raumtemperatur Öle, Harze oder überwiegend kristalline Feststoffe, die sich durch sehr wertvolle biozide Eigenschaften auszeichnen. Sie lassen sich beispielsweise auf dem Agrarsektor oder verwandten Gebieten präventiv und kurativ zur Bekämpfung phytopathogener Schädlinge, wie z.B. Pilzen oder Bakterien, einsetzen. Die erfindungsgemässen Wirkstoffe der Formel I zeichnen sich in weiten Anwendungskonzentrationen durch eine hohe biozide Aktivität und grosse Wirkungsbreite aus und können problemlos insbesondere im Agrarbereich eingesetzt werden.

Folgende Wirkstoffgruppen sind aufgrund ihrer ausgeprägten bioziden, insbesondere pflanzenfungiziden Aktivität bevorzugt:

Gruppe Ia: Verbindungen der Formel I, worin $R_1$, $R_2$, $R_3$ und $R_4$ die unter Formel I angegebenen Bedeutungen haben und $R_5$, $R_6$ und $R_7$ unabhängig voneinander für Wasserstoff, Halogen, Nitro, Cyano, $C_1$–$C_6$-Alkyl, Cyclopentyl, Cyclohexyl, $C_1$–$C_3$-Alkylthio, $C_1$–$C_3$-Alkylsulfonyl, $C_1$–$C_3$-Alkylsulfoxyl, $C_3$–$C_4$-Alkenyl, Propargyl, durch Halogen, Cyano und/oder $C_1$–$C_3$-Alkoxy substituiertes $C_1$–$C_3$-Alkyl, $C_1$–$C_6$-Alkoxy, dessen Alkylteil durch ein bis 2 einzelne Sauerstoffatome unterbrochen ist oder für die Gruppe $Q-(E)_n-(X)_m-$ stehen, worin n für 0 oder 1 und m für 0 oder 1 steht, Q für ein unsubstituiertes oder durch Halogen, Nitro, Methyl, $CF_3$ oder Methoxy substituiertes Phenyl oder eine Pyridylgruppe steht, E für eine Methylenbrücke steht und X Sauerstoff oder Schwefel bedeutet.

Gruppe Ib: Verbindungen der Formel I, worin $R_1$, $R_2$ und $R_3$ die unter Formel I angegebenen Bedeutungen haben, $R_4$ für Wasserstoff steht und $R_5$, $R_6$ und $R_7$ unabhängig voneinander für Wasserstoff, Halogen, Nitro, Cyano, $C_1$–$C_6$-Alkyl, Cyclopentyl, Cyclohexyl, $C_1$–$C_3$-Alkylthio, $C_1$–$C_3$-Alkylsulfonyl, $C_1$–$C_3$-Alkylsulfoxyl, $C_3$–$C_4$-Alkenyl, Propargyl, durch Halogen, Cyano und/oder $C_1$–$C_3$-Alkoxy substituiertes $C_1$–$C_3$-Alkyl, $C_1$–$C_6$-Alkoxy, deren Alkylteil durch ein bis 2 einzelne Sauerstoffatome unterbrochen ist oder für die Gruppe $Q-(E)_n-(X)_m-$ stehen, worin n für 0 oder 1 und m für 0 oder 1 steht, Q für ein unsubstituiertes oder durch Halogen, Nitro, Methyl, $CF_3$ oder Methoxy substituiertes Phenyl oder eine Pyridylgruppe steht, E für eine Methylenbrücke steht und X Sauerstoff oder Schwefel bedeutet.

Gruppe Ic: Verbindungen der Formel I, worin $R_1$ für $NO_2$ oder $CF_3$ steht; $R_2$ für $NO_2$ oder $CF_3$ steht; mit der Massgabe, dass nur $R_1$ oder $R_2$ $NO_2$ sein kann; $R_3$ Chlor bedeutet; $R_4$ für Wasserstoff steht; $R_6$ Wasserstoff bedeutet und $R_5$ und $R_7$ unabhängig voneinander für Wasserstoff, Fluor, Chlor, Brom, $C_1$–$C_3$-Alkyl, $C_1$–$C_3$-Haloalkyl, $C_1$–$C_3$-Alkoxy, $C_1$–$C_3$-Haloalkoxy, unsubstituiertes oder durch Halogen substituiertes Phenoxy, $OCH_2OCH_3$, $OC_2H_4OCH_3$, $OCH_2OC_2H_5$, $OC_2H_4OC_2H_5$, $OC_2H_4OC_2H_4OC_2H_5$ oder –S–(2-Pyridyl) stehen.

Gruppe Id: Verbindungen der Formel I, worin $R_1$ für $NO_2$ oder $CF_3$ steht; $R_2$ für $NO_2$ oder $CF_3$ steht; mit der Massgabe, dass nur $R_1$ oder $R_2$ $NO_2$ sein kann; $R_3$ Halogen bedeutet; $R_4$ für Wasserstoff oder die Gruppe –C(O)R′ steht, wobei R′ unsubstituiertes oder durch Halogen, $C_1$–$C_3$-Alkoxy oder $C_1$–$C_3$-Alkylthio substituiertes $C_1$–$C_4$-Alkyl bedeutet; $R_5$, $R_6$ und $R_7$ unabhängig voneinander für Wasserstoff, Halogen, Nitro, Cyano, Rhodano, $C_1$–$C_{12}$-Alkyl, $C_3$–$C_8$-Cycloalkyl, $C_1$–$C_8$-Alkylthio, $C_1$–$C_6$-Alkylsulfonyl, $C_1$–$C_6$-Alkylsulfoxyl, $C_3$–$C_6$-Alkenyl, $C_3$–$C_6$-Haloalkenyl, $C_3$–$C_6$-Alkinyl, $C_3$–$C_6$-Haloalkinyl, durch Halogen, Cyano und/oder $C_1$–$C_4$-Alkoxy substituiertes $C_1$–$C_8$-Alkyl, unsubstituiertes oder durch Halogen substituiertes $C_1$–$C_8$-Alkoxy, dessen Alkylteil gegebenenfalls durch ein oder mehrere einzelne Sauerstoffatome unterbrochen ist oder für die Gruppe $Q-(E)_n-(X)_m$ stehen, wobei n für 0 oder 1 steht, m für 0 oder 1 steht, Q für ein unsubstituiertes oder durch Halogen, Nitro, $C_1$–$C_3$-Alkyl, $CF_3$ und/oder $C_1$–$C_3$-Alkoxy substituiertes Phenyl oder einen gesättigten oder ungesättigten heterocyclischen Rest mit einem oder mehreren Heteroatomen steht, E eine $C_1$–$C_3$-Alkylenbrücke bedeutet und X für Sauerstoff oder Schwefel steht.

Besonders bevorzugte Einzelsubstanzen sind z.B.:

N-(3′-Chlor-2′,6′-dinitro-4′-trifluormethylphenyl)-2-amino-4,6-dichlorpyrimidin (Verb. Nr. 1.1),

N-(3′-Chlor-2′,6′-dinitro-4′-trifluormethylphenyl)-2-amino-4-chlor-6-trichlormethylpyrimidin (1.11),

N-(3′-Chlor-2′,6′-dinitro-4′-trifluormethylphenyl)-2-amino-4,6-dimethylpyrimidin (1.15),

N-(3′-Chlor-2′,6′-dinitro-4′-trifluormethylphenyl)-2-amino-4-trichlormethyl-6-(methoxyethoxy)pyrimidin (1.6),

N-(3′-Chlor-2′,6′-dinitro-4′-trifluormethylphenyl)-2-amino-4-methoxy-6-(2-pyridylthio)-pyrimidin (1.2),

N-(3′-Chlor-2′,6′-dinitro-4′-trifluormethylphenyl)-2-amino-4-trichlormethyl-6-(ethoxyethoxyethoxy)pyrimidin (1.4),

N-(3′-Chlor-2′,6′-dinitro-4′-trifluormethylphenyl)-2-amino-4-methoxy-6-(4″-chlorphenoxy)-pyrimidin (1.8).

Die Verbindungen der Formel I werden erfindungsgemäss dadurch hergestellt, dass man eine Verbindung der Formel II

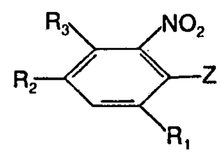
(II)

in Gegenwart einer Base mit einem Pyrimidin-Derivat der Formel III

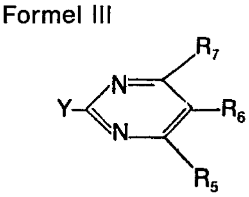
(III)

zu einer Verbindung der Formel I′

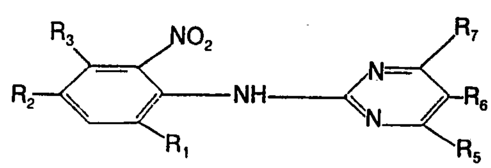
(I′)

umsetzt, und letztere zur Herstellung N-acylierter Derivate mit einem reaktionsfähigen Derivat der Carbonsäure IV

$$R_4COOH \quad (IV)$$

N-acyliert, wobei die Substituenten $R_1$ bis $R_7$ die unter Formel I angegebenen Bedeutungen haben

und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

Für die Herstellung der Verbindungen der Formel I bzw. I' sind folgende Reaktionsbedingungen vorteilhaft:

Die N-Alkylierung von (II) mit (III) zu (I'), sowie die N-Acylierung von (I') mit (IV) zu (I) erfolgen unter Halogenwasserstoffabspaltung. Die Reaktionstemperaturen liegen bei der N-Alkylierung zwischen –20° und 150 °C, vorzugsweise –20° und +30 °C, bei der N-Acylierung zwischen 0° und +180 °C, vorzugsweise 0° und 150 °C bzw. am Siedepunkt des Lösungsmittels bzw. Lösungsmittelgemisches. In beiden Fällen ist die Verwendung von säurebindenden Mitteln bzw. Kondensationsmitteln vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Entstehender Halogenwasserstoff kann in manchen Fällen auch mittels Durchleiten von Inertgas, z.B. Stickstoff aus dem Reaktionsgemisch vertrieben werden.

Die Reaktionen können in Anwesenheit von reaktionsinerten Lösungs- oder Verdünnungsmitteln durchgeführt werden. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann das Acylierungs- oder Alkylierungsmittel selbst als Lösungsmittel dienen.

Die Gegenwart eines Reaktionskatalysators wie Dimethylformamid kann von Vorteil sein.

Die Reaktion (II) und (III) kann auch in einem wässrigen Zweiphasensystem nach dem allgemein bekannten Prinzip der Phasentransferkatalyse durchgeführt werden.

Für die organische, mit Wasser nicht mischbare Phase kommen dabei z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw. Beispiele geeigneter Phasentransfer-Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw. Als Phasentransfer-Katalysatoren kommen auch Phosphonium-Salze in Betracht. Die Reaktionstemperaturen liegen im allgemeinen zwischen –30° und 130 °C, bzw. am Siedepunkt des Lösungsmittels oder Lösungsmittelgemisches.

Im übrigen kann bei der Herstellung aller hierin genannten Ausgang-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Das beschriebene Herstellungsverfahren ist, einschliesslich aller Teilschritte, ein wichtiger Bestandteil der vorliegenden Erfindung.

Es wurde überraschend festgestellt, dass Verbindungen der Formel I ein für praktische Bedürfnisse sehr günstiges biozides Spektrum gegen Pilze und Bakterien insbesondere gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen und Insekten verschont bleiben. Ferner können Akarina und unerwünschte Pflanzenarten erfolgreich mit den Wirkstoffen der Formel I bekämpft werden.

Als Mikrobizide sind die Wirkstoffe der Formel I z.B. gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); insbesondere wirken sie gegen die Klasse der Ascomyceten (z.B. Venturia, Podosphaera, Erysiphe, Monilinia, Uncinula). Überdies

wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft somit auch Schädlingsbekämpfungsmittel sowie deren Verwendung auf den Agrarsektor oder verwandten Gebieten.

Darüberhinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für den hierin offenbarten mikrobiziden Einsatz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt

werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B.
Phosphatidylethanolamin, Phosphatidylserin,
Phosphatidylcholin, Sphingomyelin,
Phosphatidylinosit, Phosphatidylglycerin,
Lysolecithin, Plasmalogene oder Cardiolipin,
die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylcholin-Mischungen. Synthetische Phospholipide sind z.B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 g Propylenglykolethergruppen enthaltenden Polyethylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:
«Mc Cutcheon's Detergents and Emulsifiers Annual» BC Publishing Corp., Ringwood New Jersey, 1981;
Helmut Stache «Tensid-Taschenbuch» Carl Hanser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei; DMSO = Dimethylsulfoxid; DMF = Dimethylformamid. Druckangaben erfolgen in Millibar mb, oder Bar b.

Beispiel H1: Herstellung von

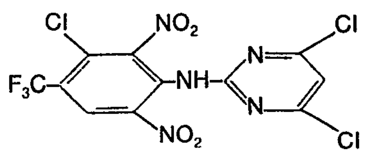

(Verb. Nr. 1.1)

N-(3′-Chlor-2′,6′-dinitro-4′-trifluormethylphenyl)-2-amino-4,6-dichlorpyrimidin

Eine Lösung von 15 Teilen 4,6-Dichlor-2-aminopyrimidin in 400 ml Tetrahydrofuran wird bei Raumtemperatur unter Rühren portionsweise mit 13,2 Teilen 85%igen, pulverisiertem Kaliumhydroxid versetzt, wobei die Temperatur innerhalb einer halben Stunde auf 23° ansteigt. Das Reaktionsgemisch wird auf 5° abgekühlt, und zu der beigen Suspension werden innerhalb von 1 Stunde 28 Teile 2,4-Dichlor-3,5-dinitro-benzotrifluorid in 80 ml Tetrahydrofuran zugetropft, wobei sich die Suspension rot verfärbt. Nach 15 stündigem Rühren bei Raumtemperatur wird das Reaktionsgemisch auf Eiswasser gegossen, mit 10 ml konzentrierter Salzsäure angesäuert und zweimal mit je 200 ml Essigsäureethylester extrahiert. Die vereinigten Extrakte werden zweimal mit je 100 ml Wasser gewaschen, über Natriumsulfat getrocknet, filtriert und eingedampft. Die zurückbleibende Kristallmasse wird aus 400 ml Isopropanol umkristallisiert; gelbe Kristalle mit Smp. 199–200°.

Analog den vorstehend beschriebenen Arbeitsweisen werden auch die nachfolgend aufgezählten Verbindungen der Formel I hergestellt:

Tabelle 1: Verbindungen der Formel

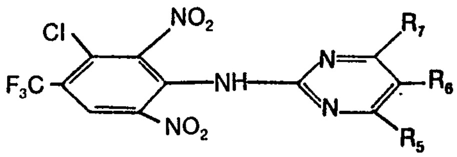

| Verb. Nr. | $R_5$ | $R_6$ | $R_7$ | physikalische Konstante [°C] |
|---|---|---|---|---|
| 1.1 | Cl | H | Cl | Smp. 198–200 |
| 1.2 | $OCH_3$ | H | –S–(2-Pyridyl) | Smp. 67–70 |
| 1.3 | H | H | H | Smp. 189–191 |
| 1.4 | $CCl_3$ | H | $-OC_2H_4OC_2H_4OC_2H_5$ | Smp. 54–59 |
| 1.5 | $CH_3$ | H | Cl | Smp. 172–174 |
| 1.6 | $CCl_3$ | H | $-OC_2H_4OCH_3$ | Smp. 130–133 |
| 1.7 | $CH_3$ | H | $OCH_3$ | Smp. 209–210 Zers. |
| 1.8 | $OCH_3$ | H | $-O-[C_6H_4Cl(4)]$ | Smp. 53–58 |
| 1.9 | Cl | H | $OCH_3$ | Smp. 180–181 |
| 1.10 | $OCH_3$ | H | $-O-[C_6H_4Cl(4)]$ | Smp. 133–137 |
| 1.11 | Cl | H | $CCl_3$ | Smp. 161–163 |
| 1.12 | $CCl_3$ | H | –S–(2-Pyridyl) | Smp. 189–192,5 |
| 1.13 | $OCH_3$ | H | $CH_2OCH_3$ | Smp. 103–106 |
| 1.14 | $CH_3$ | H | $OCHF_2$ | Smp. 147–148 |
| 1.15 | $CH_3$ | H | $CH_3$ | Smp. 155–156 |
| 1.16 | $OCH_3$ | H | $OCH_3$ | Smp. 169–171 |
| 1.17 | $OCH_3$ | H | $SC_4H_9$–n | Smp. 103–104 |
| 1.18 | $CH_3$ | H | H | Smp. 164–165 |
| 1.19 | $CF_3$ | $CH_3$ | H | Smp. 126–129 |
| 1.20 | $CF_3$ | H | $CH_3$ | Smp. 145–147 |
| 1.21 | $SCH_3$ | CN | H | |
| 1.22 | $OCH_3$ | CN | H | Smp. 152–158 |
| 1.23 | $C_2H_5$ | H | H | |
| 1.24 | H | $OC_4H_9$–i | H | |
| 1.25 | $OCH_2OCH_3$ | H | $OCH_2OCH_3$ | |
| 1.26 | Cl | J | H | |
| 1.27 | J | H | J | |

Tabelle 1: (Fortsetzung)

| Verb. Nr. | $R_5$ | $R_6$ | $R_7$ | physikalische Konstante [°C] |
|---|---|---|---|---|
| 1.28 | $CH_3$ | CN | $SO_2CH_3$ | |
| 1.29 | $OCH_3$ | J | $CH_2F$ | ab 87° Zers. |
| 1.30 | $C_6H_5$ | H | H | |
| 1.31 | H | $OCH_3$ | H | |
| 1.32 | $OC_2H_5$ | H | $CH_2F$ | Smp. 118–120 |
| 1.33 | $CH_3$ | $NO_2$ | $SO_2CH_3$ | |
| 1.34 | $C_4H_9$–n | $NO_2$ | H | |
| 1.35 | $OCH_3$ | H | $OC_2H_5$ | |
| 1.36 | $CH_3$ | H | $C_3H_7$–n | |
| 1.37 | Br | H | H | |
| 1.38 | Cl | F | H | |
| 1.39 | Cl | H | $S(O)CH_3$ | |
| 1.40 | H | O–$C_6H_5$ | H | |
| 1.41 | Cl | H | $C_6H_{13}$–n | |
| 1.42 | H | $OC_3H_7$–n | H | |
| 1.43 | $OC_2H_5$ | H | H | |
| 1.44 | $OCH_3$ | H | $CH_2OC_2H_5$ | Smp. 104–106 |
| 1.45 | $OC_4H_9$–n | H | $OC_4H_9$–n | |
| 1.46 | Cl | H | $SO_2CH_3$ | |
| 1.47 | Cl | $OCH_3$ | H | |
| 1.48 | $SC_2H_5$ | H | $SC_2H_5$ | |
| 1.49 | $CH_3$ | H | $SC_6H_5$ | |
| 1.50 | $CH_2OCH_3$ | H | H | |
| 1.51 | $OCH_3$ | $NO_2$ | H | |
| 1.52 | Cl | H | $CH_2Cl$ | Harz |
| 1.53 | Cl | $OC_6H_5$ | H | |
| 1.54 | Cl | CN | $OCH_3$ | Smp. 211–212 |
| 1.55 | $OCH_3$ | Cl | H | |
| 1.56 | Cl | $S(O)CH_3$ | H | |
| 1.57 | H | $SCH_2$–$C_6H_5$ | H | |
| 1.58 | $C_6H_5$ | $CH_3$ | H | |
| 1.59 | $SCH_3$ | H | H | |
| 1.60 | $OC_2H_5$ | $NO_2$ | H | |
| 1.61 | H | $CH_2$–$C_6H_5$ | H | |
| 1.62 | Cl | H | $C_2H_5$ | Smp. 147–148 |
| 1.63 | $OCH_3$ | H | $CH_2F$ | Smp. 111–112 |
| 1.64 | Cl | H | $OC_2H_5$ | |
| 1.65 | $CH_2OC_2H_5$ | $OC_2H_5$ | H | |
| 1.66 | H | $SCH_3$ | H | |
| 1.67 | $OCH_3$ | J | H | |
| 1.68 | Cl | $SO_2CH_3$ | H | |
| 1.69 | $OCH_3$ | F | H | |
| 1.70 | Cl | H | SCN | |
| 1.71 | $C_2H_5$ | H | Cl | Smp. 147–148 |
| 1.72 | Cl | $OC_2H_5$ | H | |
| 1.73 | $SO_2C_2H_5$ | H | H | |
| 1.74 | Cl | H | $CH_2OCH_3$ | |
| 1.75 | Cl | H | $OCH_2CH{=}CH_2$ | Smp. 97–98 |
| 1.76 | Cl | $NO_2$ | $CH_3$ | |
| 1.77 | H | $C_6H_5$ | H | |
| 1.78 | $CH_3$ | $NO_2$ | $CH_3$ | |
| 1.79 | $CH_3$ | $C_2H_5$ | H | |
| 1.80 | Br | H | $CH_3$ | |
| 1.81 | $CCl_3$ | H | $CCl_3$ | |
| 1.82 | H | Cl | H | |
| 1.83 | $CH_3$ | Br | H | |
| 1.84 | $OC_4H_9$–n | H | $CH_3$ | |
| 1.85 | Cl | $CH_3$ | H | |
| 1.86 | $CH_3$ | Cl | H | |
| 1.87 | H | $NO_2$ | H | semikristallin |
| 1.88 | Cl | Br | Cl | semikristallin |

Tabelle 1: (Fortsetzung)

| Verb. Nr. | $R_5$ | $R_6$ | $R_7$ | physikalische Konstante [°C] |
|---|---|---|---|---|
| 1.89 | $OCH_2-C_6H_5$ | H | F | |
| 1.90 | Cl | H | $OC_3H_7$-i | |
| 1.91 | $CH_3$ | Cl | $CH_3$ | |
| 1.92 | H | $S-C_6H_5$ | H | |
| 1.93 | $CH_2Br$ | Br | $CH_2Br$ | |
| 1.94 | $C_2H_5$ | $CH_3$ | H | |
| 1.95 | $CH_3$ | CN | H | |
| 1.96 | F | H | F | |
| 1.97 | H | F | H | |
| 1.98 | Cl | $CH_2-C_6H_5$ | H | |
| 1.99 | $C_6H_5$ | CN | H | |
| 1.100 | Cl | $CH_2C_6H_5$ | Cl | |
| 1.101 | Cl | $SCH_3$ | H | |
| 1.102 | Cl | $C_6H_5$ | Cl | |
| 1.103 | $CH_3$ | $NO_2$ | $SCH_3$ | |
| 1.104 | Cl | $CH_2CH_2Cl$ | $CH_3$ | |
| 1.105 | $CH_3$ | Br | $C_6H_5$ | |
| 1.106 | Cl | $C_2H_5$ | Cl | |
| 1.107 | Cl | Br | $CH_3$ | |
| 1.108 | $CH_3$ | J | $CH_3$ | |
| 1.109 | Cl | $CH_3$ | $CH_3$ | |
| 1.110 | $CH_3$ | Br | $CH_3$ | |
| 1.111 | Cl | $C_2H_5$ | $CH_3$ | |
| 1.112 | Cl | $CH_3$ | Cl | |
| 1.113 | Cl | $C_4H_9$-n | Cl | |
| 1.114 | Cl | H | $OCH_2C\equiv CH$ | Smp. 129–130 |
| 1.115 | Cl | $CH_2C\equiv CH$ | $C_6H_5$ | |
| 1.116 | $OCH_3$ | $CH_3$ | $CH_3$ | |
| 1.117 | Cl | $CH_2CH_2CN$ | $CH_3$ | |
| 1.118 | Cl | $OCH_3$ | $CH_2OCH_3$ | |
| 1.119 | Cl | Cl | $CH_3$ | |
| 1.120 | $OCH_3$ | $C_2H_5$ | $OCH_3$ | |
| 1.121 | $CH_3$ | Br | $C_3H_7$-n | |
| 1.122 | Cl | $OC_2H_5$ | Cl | |
| 1.123 | $OCH_3$ | $OCH_3$ | $CH_3$ | |
| 1.124 | $OC_2H_5$ | $OC_2H_5$ | $OC_2H_5$ | |
| 1.125 | $CH_3$ | CN | $SCH_3$ | |
| 1.126 | Cl | CN | $CH_3$ | |
| 1.127 | $CH_3$ | Br | $SCH_3$ | |
| 1.128 | Cl | $C_6H_4Cl(4)$ | Cl | |
| 1.129 | Cl | $OC_2H_5$ | $CH_2OC_2H_5$ | |
| 1.130 | $CH_3$ | Br | $OC_6H_5$ | |
| 1.131 | $OCH_3$ | $CH_3$ | $OCH_3$ | |
| 1.132 | $SCH_3$ | $C_6H_5$ | $SCH_3$ | |
| 1.133 | Cl | $OCH_3$ | Cl | |
| 1.134 | $OCH_3$ | $OCH_3$ | $CH_2OCH_3$ | |
| 1.135 | Cl | $CH_2-C_6H_5$ | $OCH_3$ | |
| 1.136 | $OCH_3$ | Br | $CH_3$ | |
| 1.137 | Cl | $C_2H_4OCH_3$ | $CH_3$ | |
| 1.138 | $OCH_3$ | $C_2H_4OC_2H_5$ | $CH_3$ | |
| 1.139 | Cl | $C_6H_4(OCH_3)(4)$ | Cl | |
| 1.140 | Cl | $C_5H_{11}$-n | Cl | |
| 1.141 | $C_5H_{11}$-n | H | H | |
| 1.142 | $CH_3$ | CN | $-S(O)CH_3$ | |
| 1.143 | Cl | H | $SCH_3$ | zähe Masse |
| 1.144 | $CH_3$ | J | $CH_3$ | Harz |
| 1.145 | Cl | H | $SCH_2CH=CH_2$ | Harz |
| 1.146 | Cl | H | $OCH_2CF_3$ | |
| 1.147 | Cl | Cl | Cl | |
| 1.148 | Cl | Br | $OCH_3$ | |
| 1.149 | Cl | Br | $OC_2H_5$ | |

Tabelle 1: (Fortsetzung)

| Verb. Nr. | $R_5$ | $R_6$ | $R_7$ | physikalische Konstante [°C] |
|---|---|---|---|---|
| 1.150 | Cl | Br | $OC_3H_7$–i | |
| 1.151 | Cl | Br | $OC_4H_9$–sek. | |
| 1.152 | Cl | Br | $OCH_2CF_3$ | |
| 1.153 | Cl | Br | $OCH_2CH_2Cl$ | |
| 1.154 | Cl | Br | $OCH_2CH_2Br$ | |
| 1.55 | Cl | Br | $OCHF_2$ | |
| 1.156 | Cl | Br | $OCH_2CH_2OCH_3$ | |
| 1.157 | Cl | Br | $SC_2H_5$ | |
| 1.158 | Cl | Br | $SC_3H_7$–i | |
| 1.159 | Cl | Br | $OC(CH_3)_2C{\equiv}CH$ | |
| 1.160 | Cl | Br | $OCH_2$–Phenyl | |
| 1.161 | Cl | Br | SCN | |
| 1.162 | Cl | Br | $OCH_2C{\equiv}CH$ | |
| 1.163 | Cl | Br | $OCH_2CH{=}CH_2$ | |

Tabelle 2: Verbindungen der Formel

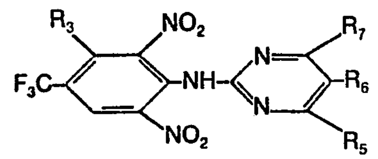

| Verb. Nr. | $R_3$ | $R_4$ | $R_6$ | $R_7$ | physikalische Konstante [°C] |
|---|---|---|---|---|---|
| 2.1 | F | Cl | H | Cl | Harz |
| 2.2 | J | Cl | H | Cl | Harz |
| 2.3 | Br | F | $CH_3$ | F | |
| 2.4 | Br | Cl | $CH_3$ | Cl | |
| 2.5 | F | Cl | $OC_2H_5$ | Cl | |
| 2.6 | J | $C_2H_5$ | H | H | |
| 2.7 | F | Cl | $CH_2$–$C_6H_5$ | Cl | |
| 2.8 | Br | $OC_2H_5$ | H | H | |
| 2.9 | Br | Cl | $NO_2$ | $SCH_3$ | |
| 2.10 | F | Cl | Cl | $CH_3$ | |
| 2.11 | Br | Cl | F | H | |
| 2.12 | F | $SCH_3$ | CN | H | |
| 2.13 | F | $OCH_3$ | CN | H | |

Tabelle 3: Verbindungen der Formel

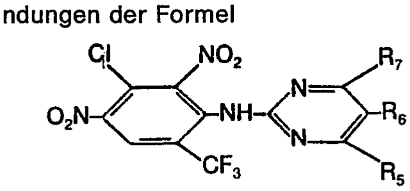

| Verb. Nr. | $R_5$ | $R_6$ | $R_7$ | physikalische Konstante [°C] |
|---|---|---|---|---|
| 3.1 | Cl | H | $OCH_2F$ | Harz |
| 3.2 | Cl | H | Cl | |
| 3.3 | $SCH_3$ | CN | H | |
| 3.4 | $OCH_3$ | CN | H | |

Tabelle 3: (Fortsetzung)

| Verb. Nr. | $R_5$ | $R_6$ | $R_7$ | physikalische Konstante [°C] |
|---|---|---|---|---|
| 3.5 | $CH_3$ | $CH_3$ | H | |
| 3.6 | $OCH_3$ | Br | H | |
| 3.7 | $OC_3H_7$–i | H | $OC_3H_7$–i | |
| 3.8 | $CH_3$ | CN | H | |
| 3.9 | $OC_2OC_2H_5$ | H | $CH_3$ | |
| 3.10 | $SC_2H_5$ | H | $CH_3$ | |
| 3.11 | $OC_2H_5$ | H | F | |
| 3.12 | $OCH_3$ | $OC_2H_5$ | H | |
| 3.13 | $OCH_3$ | J | $CH_2F$ | Harz |
| 3.14 | $CH_3$ | CN | $S(O)CH_3$ | |
| 3.15 | $OC_2H_5$ | H | $CH_3$ | |
| 3.16 | $C_4H_9$–i | H | H | |
| 3.17 | Cl | $CH_3$ | H | |
| 3.18 | Cl | H | H | |
| 3.19 | $C_6H_5$ | CN | H | |
| 3.20 | H | $CH_2$–$C_6H_5$ | H | |
| 3.21 | $CH_3$ | CN | $SO_2CH_3$ | |
| 3.22 | $C_3H_7$–n | H | H | |
| 3.23 | Cl | $OCH_3$ | H | |
| 3.24 | $CH_2OCH_3$ | H | $CH_3$ | |
| 3.25 | $CHBr_2$ | Br | H | |
| 3.26 | $OCH_3$ | $OCH_3$ | H | |
| 3.27 | Cl | H | $SC_2H_5$ | |
| 3.28 | $CH_3$ | $NO_2$ | $SO_2CH_3$ | |
| 3.29 | $SC_3H_7$–i | H | $CH_3$ | |
| 3.30 | Cl | F | H | |
| 3.31 | $OC_2H_5$ | $OCH_3$ | H | |
| 3.32 | $CH_3$ | H | $SC_3H_7$–n | |
| 3.33 | $OCH_3$ | H | H | |
| 3.34 | $CH_3$ | $C_2H_4OC_2H_5$ | H | |
| 3.35 | Cl | H | $SO_2CH_3$ | |
| 3.36 | $OC_2H_5$ | $NO_2$ | H | |
| 3.37 | $OCH_3$ | F | H | |
| 3.38 | $CH_3$ | H | $SCH_3$ | |
| 3.39 | $C_4H_9$–n | H | $CH_3$ | |
| 3.40 | $SC_2H_5$ | H | H | |
| 3.41 | Cl | H | $OC_2H_5$ | |
| 3.42 | Cl | $SO_2CH_3$ | H | |
| 3.43 | $C_3H_7$–i | H | H | |
| 3.44 | H | $C_4H_9$–n | H | |
| 3.45 | $CH_2OCH_3$ | $OCH_3$ | H | |
| 3.46 | H | CN | H | |
| 3.47 | Cl | $OC_2H_5$ | H | |
| 3.48 | $SO_2C_2H_5$ | H | H | |
| 3.49 | $CH_3$ | H | O–$C_6H_5$ | |
| 3.50 | $OCH_2$–$C_6H_5$ | H | F | |
| 3.51 | Cl | H | $SCH_3$ | |
| 3.52 | F | H | F | |
| 3.53 | Cl | $CH_2$–$C_6H_5$ | $C_6H_5$ | |
| 3.54 | H | Br | H | |
| 3.55 | Cl | $SCH_3$ | H | |
| 3.56 | $CCl_3$ | H | $CCl_3$ | |
| 3.57 | H | $C_3H_7$–i | H | |
| 3.58 | $CH_3$ | H | $CF_3$ | |
| 3.59 | Cl | S–$C_6H_5$ | H | |
| 3.60 | $OC_3H_7$–i | H | $CH_3$ | |
| 3.61 | Cl | H | SCN | |
| 3.62 | $CH_2$–$C_6H_5$ | H | $CH_3$ | |
| 3.63 | Cl | $CH_2$–$C_6H_5$ | Cl | |
| 3.64 | $SCH_2$–$C_6H_5$ | H | $CH_3$ | |
| 3.65 | Cl | Br | Cl | |

Tabelle 3: (Fortsetzung)

| Verb. Nr. | $R_5$ | $R_6$ | $R_7$ | physikalische Konstante [°C] |
|---|---|---|---|---|
| 3.66 | Cl | $CH_2-C_6H_5$ | $CH_3$ | |
| 3.67 | $OC_2H_5$ | H | $CH_2F$ | Harz |
| 3.68 | Cl | $C_6H_5$ | Cl | |
| 3.69 | Cl | Cl | Cl | |
| 3.70 | H | $CH_3$ | H | |
| 3.71 | $SCH_2-C_6H_5$ | H | $SCH_2-C_6H_5$ | |
| 3.72 | $OCH_3$ | H | $CH_2OC_2H_5$ | Harz |
| 3.73 | Cl | $CH_3$ | Cl | |
| 3.74 | H | $OC_4H_9$ | H | |
| 3.75 | Cl | $OC_2H_5$ | Cl | |
| 3.76 | Cl | Cl | $CH_3$ | |
| 3.77 | Cl | $C_5H_9$–n | $CH_3$ | |
| 3.78 | $SCH_3$ | H | $SCH_3$ | |
| 3.79 | Cl | $C_4H_9$–n | Cl | |
| 3.80 | H | $OC_4H_9$–sek. | H | |
| 3.81 | Cl | H | $CH_2Cl$ | |
| 3.82 | $CH_3$ | $C_6H_5$ | $CH_3$ | |
| 3.83 | $CH_3$ | $CH_3$ | $CH_3$ | |
| 3.84 | H | Cyclohexyl | H | |
| 3.85 | $SC_3H_7$–n | H | $SC_3H_7$–n | |
| 3.86 | H | $OC_2H_5$ | H | |
| 3.87 | Cl | CN | $OCH_3$ | |
| 3.88 | $OCH_3$ | H | $CH_2F$ | |
| 3.89 | $OCH_3$ | $OCH_3$ | $OCH_3$ | |
| 3.90 | $C_2H_5$ | H | Cl | |
| 3.91 | CN | H | $CH_3$ | |
| 3.92 | Cl | $NO_2$ | $OCH_3$ | |
| 3.93 | H | $OC_2H_4OCH_3$ | H | |
| 3.94 | Cl | H | $C_6H_5$ | |
| 3.95 | $CH_3$ | $CH_2-CH=CH_2$ | $CH_3$ | |
| 3.96 | $C_6H_5$ | H | $C_6H_5$ | |
| 3.97 | $SC_4H_9$–n | H | $CH_3$ | |
| 3.98 | $CH_3$ | H | $C_6H_5$ | |
| 3.99 | Br | H | Br | |
| 3.100 | $OC_2H_5$ | H | $OC_2H_5$ | |
| 3.101 | H | $CH_2OCH_3$ | H | |
| 3.102 | Cl | $OCH_3$ | Cl | |
| 3.103 | $OCH_3$ | H | $SCH_3$ | |
| 3.104 | Cl | $OCH_3$ | $CH_3$ | |
| 3.105 | Cl | $C_3H_7$–n | $C_6H_5$ | |
| 3.106 | Cl | $C_6H_3Cl_2(2,4)$ | Cl | |
| 3.107 | Cl | H | $SC_3H_7$–n | |
| 3.108 | Cl | $C_6H_4(CH_3)(4)$ | Cl | |
| 3.109 | Cl | Br | $CH_3$ | |
| 3.110 | Cl | $C_2H_5$ | Cl | |
| 3.111 | Cl | $C_6H_4(OCH_3)(4)$ | Cl | |
| 3.112 | Cl | $CH_3$ | $C_6H_5$ | |
| 3.113 | $C_6H_4(CF_3)(4)$ | CN | H | |
| 3.114 | Cl | $C_6H_4(NO_2)(4)$ | Cl | |
| 3.115 | Cl | H | $SCH_3$ | |
| 3.116 | Cl | H | $OCH_2CH=CH_2$ | Harz |
| 3.117 | $C_6H_{13}$–n | H | H | |
| 3.118 | Cl | H | $OCH_2C\equiv CH$ | |
| 3.119 | Cl | H | $SCH_3$ | |
| 3.120 | Cl | Cl | $OCH_3$ | |
| 3.121 | Cl | Cl | $OC_2H_5$ | |
| 3.122 | Cl | Cl | $OC_4H_9$–sek. | |
| 3.123 | Cl | Cl | $OC_4H_9$–tert. | |
| 3.124 | Cl | Cl | $OCH_2CF_3$ | |
| 3.125 | Cl | Cl | $OCHF_2$ | |
| 3.126 | Cl | Cl | $OCH_2CH_2Cl$ | |

Tabelle 3: (Fortsetzung)

| Verb. Nr. | $R_5$ | $R_6$ | $R_7$ | physikalische Konstante [°C] |
|---|---|---|---|---|
| 3.127 | Cl | Cl | $OCH_2CH_2OC_2H_5$ | |
| 3.128 | Cl | Cl | $OC(CH_3)_2{\equiv}CH$ | semikristallin |
| 3.129 | Cl | Cl | $OCH_2$-Phenyl | |
| 3.130 | Cl | Cl | SCN | |
| 3.131 | Cl | Cl | $SC_2H_5$ | |
| 3.132 | Cl | Cl | $SC_3H_7$-i | |
| 3.132 | Cl | Cl | $OCH_2C{\equiv}CH$ | |
| 3.133 | Cl | Cl | $OCH_2{=}CH_2$ | |

Tabelle 4: Verbindungen der Formel

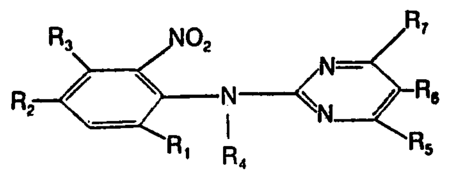

| Verb. Nr. | $R_1$ | $R_2$ | $R_3$ | $R_4$ | $R_5$ | $R_6$ | $R_7$ | physikalische Konstante [°C] |
|---|---|---|---|---|---|---|---|---|
| 5.1 | $NO_2$ | $CF_3$ | Cl | $C(O)CH_3$ | Cl | H | Cl | |
| 5.2 | $CF_3$ | $NO_2$ | Cl | $C(O)CH_3$ | Cl | H | Cl | |
| 5.3 | $NO_2$ | $CF_3$ | Cl | $C(O)CH_2Cl$ | Cl | $C_4H_9$-n | Cl | |
| 5.4 | $NO_2$ | $CF_3$ | Cl | $C(O)CH_2OCH_3$ | Cl | $CH_3$ | Cl | |
| 5.5 | $NO_2$ | $CF_3$ | Cl | $C(O)CH_3$ | Cl | $OC_2H_5$ | Cl | |
| 5.6 | $CF_3$ | $NO_2$ | Cl | $C(O)CH_2OC_2H_5$ | Cl | $CH_3$ | Cl | |
| 5.7 | $CF_3$ | $NO_3$ | Cl | $C(O)CH_2OCH_3$ | Cl | CN | $CH_3$ | |
| 5.8 | $NO_2$ | $CF_3$ | Cl | $C(O)CH_3$ | $SCH_3$ | CN | H | |
| 5.9 | $NO_2$ | $CF_3$ | Cl | $C(O)CH_3$ | $OCH_3$ | CN | H | |

sowie die Verbindung

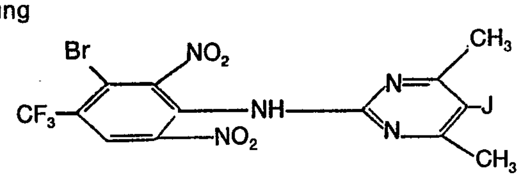

zähe Masse
Verb. Nr. 6.1

Formulierungsbeispiele für flüssige Wirkstoffe der Formel I
(% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | – | – |
| Tributylphenoyl-polyethylenglykolether (30 Mol Ethylenoxid) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | – | – | – |
| Polyethylenglykol M G 400 | – | 70% | – | – |
| N-Methyl-2-pyrrolidon | – | 20% | – | – |
| Epoxydiertes Kokosnussöl | – | – | 1% | 5% |
| Benzin (Siedegrenzen 160–190 °C) | | | | |
| (MG = Molekulargewicht) | – | – | 94% | – |

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | – |
| Kaolin | – | 90% |

Durch inniges Vermischen auf Trägerstoffe mit dem Wirkstoff erhält man gebrauchsfertige Stäubemittel.

Formulierungsbeispiele für feste Wirkstoffe der Formel I
(% = Gewichtsprozent)

| F5. Spritzpulver | a) | b) | b) |
|---|---|---|---|
| Wirkstoff aus den Tabellen | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyethylenglykolether | | | |
| (7–8 Mol Ethylenoxid) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff aus den Tabellen | 10% |
| Octylphenolpolyethylenglykolether (4–5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | – |
| Kaolin | – | 92% |

Man erhält anwendungsfertige Stäubemittel, in dem der Wirkstoff mit dem Träger vermischt auf einer geeigneten Mühle vermahlen wird.

| F8. Extruder Granulat | |
|---|---|
| Wirkstoff aus den Tabellen | 10% |
| N-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| F9. Umhüllungs-Granulat | |
|---|---|
| Wirkstoff aus den Tabellen | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| F10. Suspensions-Konzentrat | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:
Beispiel B1:
Wirkung gegen Puccinia graminis auf Weizen
a) Residual-protektive Wirkung

Weizenpflanzen wurden 6 Tage nach der Aussaat mit einer aus Spritzpulver des Wirkstoffes hergestellten Spritzbrühe (0,02% Aktivsubstanz) besprüht. Nach 24 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus der Tabelle zeigten gegen Puccinia-Pilze eine sehr gute Wirkung. Unbehandelte aber infizierte Kontrollpflanzen zeigten einen Puccinia-Befall von 100%. Unter anderem hemmten die Verbindungen 1.1 bis 1.19, 1.22, 1.32, 1.44, 1.52, 1.54, 1.62, 1.63, 1.71, 1.75, 1.88, 1.114, 1.143, 1.144, 1.145, 2.2, 3.1, 3.13, 3.72, 3.116 und 3.128 den Puccinia-Befall auf 0 bis 15%.

Beispiel B2:
Wirkung gegen Cersopora arachidicola auf Erdnusspflanzen
Residual-protektive Wirkung

10–15 cm hohe Erdnusspflanzen wurden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen behandelt wurden, einen stark reduzierten Cercospora-Befall. So verhinderten die Verbindungen 1.1 bis 1.19, 1.22, 1.32, 1.44, 1.52, 1.54, 1.62, 1.63, 1.71, 1.75, 1.88, 1.114, 1.143, 1.144, 1.145, 2.2, 3.1, 3.13, 3.72, 3.116 und 3.128 in obigen Versuchen das Auftreten von Flekken fast vollständig (0–10%).

Beispiel B3:
Wirkung gegen Erysiphae graminis auf Gerste
a) Residual-protektive Wirkung

Ca. 8 cm hohe Gerstenpflanzen wurden mit einer aus Spritzpulver des Wirkstoffes hergestellten Spritzbrühe (0,002% Aktivsubstanz) besprüht. Nach 3–4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100%. Unter anderen Verbindungen aus den Tabellen 1 und 2 hemmten den Pilzbefall bei Gerste auf weniger als 30%.

Beispiel B4:
Residual-protektive Wirkung gegen Venturia inaequalis auf Apfeltrieben

Apfelstecklinge mit 10–20 cm langen Frischtrieben wurden mit einer aus Spritzpulver des Wirkstoffes hergestellten Spritzbrühe (0,006% Aktivsubstanz) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24 °C aufgestellt. Der Schorfbefall wurde 15 Tage nach der Infektion beurteilt. Verbindungen aus den Tabellen 1 und 2 hemmten den Krankheitsbefall auf weniger als 25%. Unbehandelte aber infizierte Kontrolltriebe wurden dagegen 100%ig befallen.

Beispiel B5:
Wirkung gegen Botrytis cinerea auf Bohnen
Residual-protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95–100% relativer Luftfeuchtigkeit und 21 °C erfolgte die Beurteilung des Pilzbefalls. Die Verbindungen aus der Tabelle hemmten in vielen Fällen die Pilzinfektion sehr stark. Bei einer Konzentration von 0,02% erwiesen sich z.B. die Verbindungen 1.1, 1.2, 1.4, 1.6, 1.8, 1.11, 1.15 und 2.2 als voll wirksam. Der Krankheitsbefall lag bei 0 bis 8%. Der Botrytis-Befall unbehandelter aber infizierter Bohnenpflanzen betrug 100%.

Beispiel B6:
Wirkung gegen Phytophthora infestans auf Tomatenpflanzen
a) Residual-protektive Wirkung

Tomatenpflanzen wurden nach 3-wöchiger Anzucht mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90–100% relativer Luftfeuchtigkeit und 20 °C.

b) Systemische Wirkung

Zu Tomatenpflanzen wurde nach 3-wöchiger Anzucht eine aus Spritzpulver des Wirkstoffes hergestellte Spritzbrühe gegossen (0,06% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90–100% relativer Luftfeuchtigkeit und 20 °C.

Unter anderen zeigten in obigen Versuchen die Verbindungen Nr. 1.1, 1.2, 1.4, 1.6, 1.8 bis 1.20, 1.22, 1.29, 1.44, 1.63, 1.75, 1.88, 1.43 bis 1.45, 2.2, 3.1 und 3.116 eine sehr gute systemische Wirkung. Gegenüber unbehandelten Kontrollpflanzen (100% Befall) bewirkten diese Verbindungen eine fast vollständige Unterdrückung des Pilzbefalls (0 bis 5%).

Beispiel B7:
Wirkung gegen Plasmapora viticola auf Reben
a) Residual-protektive Wirkung

Im 4–5 Blattstadium wurden Rebensämlinge mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Nach einer Inkubation während 6 Tagen bei 95–100% relativer Luftfeuchtigkeit und 20 °C wurde der Pilzbefall beurteilt.

b) Residual-kurative Wirkung

Im 4–5 Blattstadium wurden Rebensämlinge mit einer Sporangiensuspension des Pilzes infiziert. Nach einer Inkubation während 24 Stunden in einer Feuchtkammer bei 95–100% relativer Luftfeuchtigkeit und 20 °C wurden die infizierten Pflanzen getrocknet und mit einer aus Spritzpulver des Wirkstoffes hergestellten Spritzbrühe (0,06% Aktivsubstanz) besprüht. Nach dem Antrocknen des Spritzbelages wurden die behandelten Pflanzen wieder in die Feuchtkammer gebracht. Die Beurteilung des Pilzbefalls erfolgte 6 Tage nach der Infektion.

Verbindungen aus den Tabellen zeigten gegen Plasmopara viticola auf Regen eine sehr gute fungizide Wirkung, insbesondere die Wirkstoffe Nr. 1.1, 1.4, 1.6, 1.8 und 1.11 bewirkten eine vollständige Unterdrückung des Pilzbefalls (0 bis 5%).

Beispiel B8:
Wirkung gegen Piricularia oryzae auf Reispflanzen
Residual-protektive Wirkung

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95–100% relativer Luftfeuchtigkeit und 24 °C wurde der Pilzbefall beurteilt.

Reispflanzen, die mit einer Spritzbrühe behandelt worden sind, die als Aktivsubstanz eine der Verbindungen 1.1, 1.4, 1.6, 1.8 und 2.2 enthielt, zeigten im Vergleich zu unbehandelten Kontrollpflanzen (100% Befall) weniger als 10% Pilzbefall.

**Patentansprüche für die Vertragsstaaten: GB, BE, DE, FR, IT, LU, NL, SE, CH, LI**

1. Verbindungen der allgemeinen Formel I

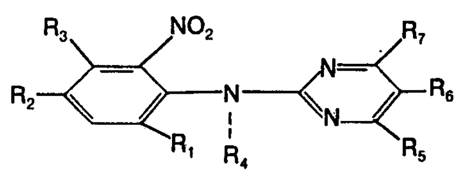
(I)

worin

$R_1$ und $R_2$ für Wasserstoff, $NO_2$ oder $CF_3$ stehen, mit der Massgabe, dass nur $R_1$ oder $R_2$ für $NO_2$ stehen kann;

$R_3$ Halogen bedeutet;

$R_4$ für Wasserstoff oder die Gruppe $-C(O)R'$ steht, wobei R′ unsubstituiertes oder durch Halogen, $C_1$–$C_3$-Alkoxy oder $C_1$–$C_3$-Alkylthio substituiertes $C_1$–$C_4$-Alkyl bedeutet;

$R_5$, $R_6$ und $R_7$ unabhängig voneinander für Wasserstoff, Halogen, Nitro, Cyano, Rhodano, $C_1$–$C_{12}$-Alkyl, $C_3$–$C_8$-Cycloalkyl, $C_1$–$C_8$-Alkylthio, $C_1$–$C_6$-Alkylsulfonyl, $C_1$–$C_6$-Alkylsulfoxyl, $C_3$–$C_6$-Alkenyl, $C_3$–$C_6$-Haloalkenyl, $C_3$–$C_6$-Alkinyl, $C_3$–$C_6$-Haloalkinyl, $C_3$–$C_6$-Alkenyloxy, $C_3$–$C_6$-Haloalkenyloxy, $C_3$–$C_6$-Alkenylthio, $C_3$–$C_6$-Alkinyloxy, $C_3$–$C_6$-Haloalkinyloxy, durch Halogen, Cyano, und/oder $C_1$–$C_4$-Alkoxy substituiertes $C_1$–$C_8$-Alkyl, unsubstituiertes oder durch Halogen substituiertes $C_1$–$C_8$-Alkoxy, dessen Alkylteil gegebenenfalls durch ein oder mehrere einzelne Sauerstoffatome unterbrochen ist oder für die Gruppe $Q-(E)_n-(X)_m$ stehen, wobei n für 0 oder 1 steht, m für 0 oder 1 steht, Q für ein

unsubstituiertes oder durch Halogen, Nitro, $C_1$–$C_3$-Alkyl, $CF_3$ und/oder $C_1$–$C_3$-Alkoxy substituiertes Phenyl oder einen gesättigten oder ungesättigten fünf- oder sechsgliedrigen Heterocyclus mit ein bis drei gleichen oder verschiedenen Heteroatomen, wie Sauerstoff, Stickstoff oder Schwefel, E eine $C_1$–$C_3$ Alkylenbrücke bedeutet und X für Sauerstoff oder Schwefel steht.

2. Verbindungen der Formel I nach Anspruch 1, dadurch gekennzeichnet, dass

$R_1$ für $NO_2$ oder $CF_3$ steht;

$R_2$ für $NO_2$ oder $CF_3$ steht; mit der Massgabe, dass nur $R_1$ oder $R_2$ $NO_2$ sein kann;

$R_3$ Halogen bedeutet;

$R_4$ für Wasserstoff oder die Gruppe –C(O)R′ steht, wobei R′ unsubstituiertes oder durch Halogen, $C_1$–$C_3$-Alkoxy oder $C_1$–$C_3$-Alkylthio substituiertes $C_1$–$C_4$-Alkyl bedeutet;

$R_5$, $R_6$ und $R_7$ unabhängig voneinander für Wasserstoff, Halogen, Nitro, Cyano, Rhodano, $C_1$–$C_{12}$-Alkyl, $C_3$–$C_8$-Cycloalkyl, $C_1$–$C_8$-Alkylthio, $C_1$–$C_6$-Alkylsulfonyl, $C_1$–$C_6$-Alkylsulfoxyl, $C_3$–$C_6$-Alkenyl, $C_3$–$C_6$-Haloalkenyl, $C_3$–$C_6$-Alkinyl, $C_3$–$C_6$-Haloalkinyl, durch Halogen, Cyano und/oder $C_1$–$C_4$-Alkoxy substituiertes $C_1$–$C_8$-Alkyl, unsubstituiertes oder durch Halogen substituiertes $C_1$–$C_8$-Alkoxy, dessen Alkylteil gegebenenfalls durch ein oder mehrere einzelne Sauerstoffatome unterbrochen ist oder für die Gruppe $Q–(E)_n–(X)_m$ stehen, wobei n für 0 oder 1 steht, m für 0 oder 1 steht, Q für ein unsubstituiertes oder durch Halogen, Nitro, $C_1$–$C_3$-Alkyl, $CF_3$ und/oder $C_1$–$C_3$-Alkoxy substituiertes Phenyl oder einen gesättigten oder ungesättigten fünf- oder sechsgliedrigen Heterocyclus mit einem bis drei Heteroatomen, wie Sauerstoff, Stickstoff oder Schwefel, steht, E eine $C_1$–$C_3$ Alkylenbrücke bedeutet und X für Sauerstoff oder Schwefel steht.

3. Verbindungen der Formel I nach Anspruch 2, dadurch gekennzeichnet, dass $R_1$, $R_2$, $R_3$ und $R_4$ die unter Formel I angegebenen Bedeutungen haben und $R_5$, $R_6$ und $R_7$ unabhängig voneinander für Wasserstoff, Halogen, Nitro, Cyano, $C_1$–$C_6$-Alkyl, Cyclopentyl, Cyclohexyl, $C_1$–$C_3$-Alkylthio, $C_1$–$C_3$-Alkylsulfonyl, $C_1$–$C_3$-Alkylsulfoxyl, $C_3$–$C_4$-Alkenyl, Propargyl, durch Halogen, Cyano und/oder $C_1$–$C_3$-Alkoxy substituiertes $C_1$–$C_3$-Alkyl, $C_1$–$C_6$-Alkoxy, dessen Alkylteil durch ein bis 2 einzelne Sauerstoffatome unterbrochen ist oder für die Gruppe $Q–(E)_n–(X)_m$ stehen, worin n für 0 oder 1 und m für 0 oder 1 steht, Q für ein unsubstituiertes oder durch Halogen, Nitro, Methyl, $CF_3$ oder Methoxy substituiertes Phenyl oder eine Pyridylgruppe steht, E für eine Methylenbrücke steht und X Sauerstoff oder Schwefel bedeutet.

4. Verbindungen der Formel I nach Anspruch 3, dadurch gekennzeichnet, dass $R_1$, $R_2$ und $R_3$ die unter Formel I angegebenen Bedeutungen haben, $R_4$ für Wasserstoff steht, und $R_5$, $R_6$ und $R_7$ unabhängig voneinander für
Wasserstoff, Halogen, Nitro, Cyano, $C_1$–$C_6$-Alkyl, Cyclopentyl, Cyclohexyl, $C_1$–$C_3$-Alkylthio, $C_1$–$C_3$-Alkylsulfonyl, $C_1$–$C_3$-Alkylsulfoxyl, $C_3$–$C_4$-Alkenyl, Propargyl, durch Halogen, Cyano und/oder $C_1$–$C_3$-Alkoxy substituiertes $C_1$–$C_3$-Alkyl, $C_1$–$C_6$-Alkoxy, dessen Alkylteil durch ein bis 2 einzelne Sauerstoffatome unterbrochen ist oder für die Gruppe $Q–(E)_n–(X)_m$ stehen, worin n für 0 oder 1 und m für 0 oder 1 steht, Q für ein unsubstituiertes oder durch Halogen, Nitro, Methyl, $CF_3$ oder Methoxy substituiertes Phenyl oder eine Pyridylgruppe steht, E für eine Methylenbrücke steht und X Sauerstoff oder Schwefel bedeutet.

5. Verbindungen der Formel I nach Anspruch 4, dadurch gekennzeichnet, dass $R_1$ für $NO_2$ oder $CF_3$ steht; mit der Massgabe, dass nur $R_1$ oder $R_2$ $NO_2$ sein kann; $R_2$ für $NO_2$ oder $CF_3$ steht; $R_3$ Chlor bedeutet; $R_4$ für Wasserstoff steht; $R_6$ Wasserstoff bedeutet und $R_5$ und $R_7$ unabhängig voneinander für Wasserstoff, Fluor, Chlor, Brom, $C_1$–$C_3$-Alkyl, $C_1$–$C_3$-Haloalkyl, $C_1$–$C_3$-Alkoxy, $C_1$–$C_3$-Haloalkoxy, unsubstituiertes oder durch Halogen substituiertes Phenoxy, $OCH_2OCH_3$, $OC_2H_4OCH_3$, $OCH_2OC_2H_5$, $OC_2H_4OC_2H_5$, $OC_2H_4OC_2H_4OC_2H_5$ oder –S–(2-Pyridyl) stehen.

6. Eine Verbindung der Formel I nach Anspruch 1, ausgewählt aus der Reihe:

N-(3′-Chlor-2′,6′-dinitro-4′-trifluormethylphenyl)-2-amino-4,6-dichlorpyrimidin,

N-(3′-Chlor-2′,6′-dinitro-4′-trifluormethylphenyl)-2-amino-4-chlor-6-trichlormethylpyrimidin,

N-(3′-Chlor-2′,6′-dinitro-4′-trifluormethylphenyl)-2-amino-4,6-dimethylpyrimidin,

N-(3′-Chlor-2′,6′-dinitro-4′-trifluormethylphenyl)-2-amino-4-trichlormethyl-6-(methoxyethoxy)pyrimidin,

N-(3′-Chlor-2′,6′-dinitro-4′-trifluormethylphenyl)-2-amino-4-methoxy-6-(2-pyridylthio)-pyrimidin,

N-(3′-Chlor-2′,6′-dinitro-4′-trifluormethylphenyl)-2-amino-4-trichlormethyl-6-(ethoxyethoxyethoxy)pyrimidin und

N-(3′-Chlor-2′,6′-dinitro-4′-trifluormethylphenyl)-2-amino-4-methoxy-6-(4″-chlorphenoxy)-pyrimidin.

7. Verfahren zur Herstellung von Verbindungen der Formel I, dadurch gekennzeichnet, dass man eine Verbindung der Formel II

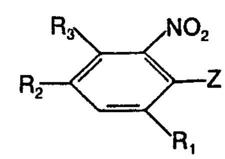

(II)

in Gegenwart einer Base mit einem Pyrimidin-Derivat der Formel III

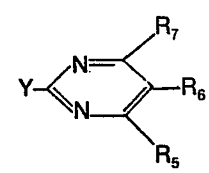

(III)

zu einer Verbindung der Formel I′

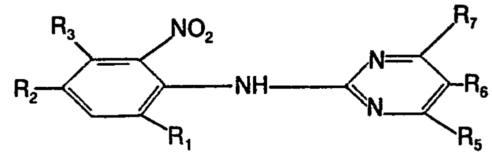

(I′)

umsetzt, und letztere zur Herstellung N-acylierter Derivate mit einem reaktionsfähigen Derivat der Carbonsäure IV

$R_4COOH$ (IV)

N-acyliert, wobei die Substituenten $R_1$ bis $R_7$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

8. Mittel zur Bekämpfung oder Verhütung eines Befalls durch phytopathogene Schädlinge, dadurch gekennzeichnet, dass es als mindestens eine aktive Komponente eine Verbindung der Formel I zusammen mit üblichen Zuschlagstoffen und Trägermaterialien enthält.

9. Mittel gemäss Anspruch 8, dadurch gekennzeichnet, dass es als mindestens eine aktive Komponente eine Verbindung der Formel I gemäss Anspruch 2 enthält.

10. Mittel zur Bekämpfung von Mikroorganismen gemäss Anspruch 8, dadurch gekennzeichnet, dass es als mindestens eine aktive Komponente eine Verbindung der Formel I gemäss einem der Ansprüche 3 bis 6 enthält.

11. Verfahren zur Bekämpfung oder Verhütung eines Befalls von Kulturpflanzen durch phytopathogene Schädlinge, dadurch gekennzeichnet, dass man eine Verbindung der Formel I auf die Pflanze oder deren Standort appliziert.

**Patentansprüche für den Vertragsstaat AT**

1. Verfahren zur Herstellung von Verbindungen der Formel I

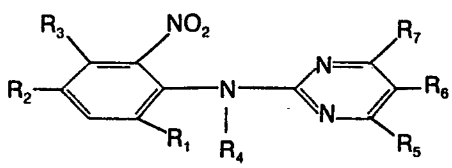

(I)

worin

$R_1$ und $R_2$ für Wasserstoff, $NO_2$ oder $CF_3$ stehen, mit der Massgabe, dass nur $R_1$ oder $R_2$ für $NO_2$ stehen kann;

$R_3$ Halogen bedeutet;

$R_4$ für Wasserstoff oder die Gruppe $-C(O)R'$ steht, wobei R' unsubstituiertes oder durch Halogen, $C_1$–$C_3$-Alkoxy oder $C_1$–$C_3$-Alkylthio substituiertes $C_1$–$C_4$-Alkyl bedeutet;

$R_5$, $R_6$ und $R_7$ unabhängig voneinander für Wasserstoff, Halogen, Nitro, Cyano, Rhodano, $C_1$–$C_{12}$-Alkyl, $C_3$–$C_8$-Cycloalkyl, $C_1$–$C_8$-Alkylthio, $C_1$–$C_6$-Alkylsulfonyl, $C_1$–$C_6$-Alkylsulfoxyl, $C_3$–$C_6$-Alkenyl, $C_3$–$C_6$-Haloalkenyl, $C_3$–$C_6$-Alkinyl, $C_3$–$C_6$-Haloalkinyl, $C_3$–$C_6$-Alkenylenoxy, $C_3$–$C_6$-Haloalkenyloxy, $C_3$–$C_6$-Alkenylthio, $C_3$–$C_6$-Alkinyloxy, $C_3$–$C_6$-Haloalkinyloxy, durch Halogen, Cyano und/oder $C_1$–$C_4$-Alkoxy substituiertes $C_1$–$C_8$-Alkyl, unsubstituiertes oder durch Halogen substituiertes $C_1$–$C_8$-Alkoxy, dessen Alkylteil gegebenenfalls durch ein oder mehrere einzelne Sauerstoffatome unterbrochen ist oder für die Gruppe $Q-(E)_n-(X)_m-$ stehen, wobei n für 0 oder 1 steht, m für 0 oder 1 steht, Q für ein unsubstituiertes oder durch Halogen, Nitro, $C_1$–$C_3$-Alkyl, $CF_3$ und/oder $C_1$–$C_3$-Alkoxy substituiertes Phenyl oder einen gesättigten oder ungesättigten fünf- oder sechsgliedrigen Heterocyclus mit ein bis drei gleichen oder verschiedenen Heteroatomen, wie Sauerstoff, Stickstoff oder Schwefel, steht, E eine $C_1$–$C_3$ Alkylenbrücke bedeutet und X für Sauerstoff oder Schwefel steht, dadurch gekennzeichnet, dass man eine Verbindung der Formel II

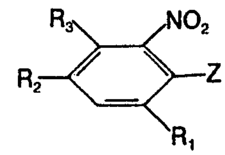

(II)

in Gegenwart einer Base mit einem Pyrimidin-Derivat der Formel III

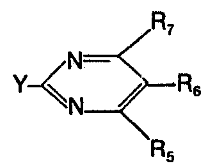

(III)

zu einer Verbindung der Formel I'

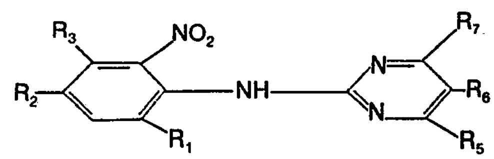

(I')

umsetzt, und letztere zur Herstellung N-acylierter Derivate mit einem reaktionsfähigen Derivat der Carbonsäure IV

$R_4COOH$ (IV)

N-acyliert, wobei die Substituenten $R_1$ bis $R_7$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

2. Verfahren nach Anspruch 1, zur Herstellung von Verbindungen der Formel I, worin

$R_1$ für $NO_2$ oder $CF_3$ steht;

$R_2$ für $NO_2$ oder $CF_3$ steht; mit der Massgabe, dass nur $R_1$ oder $R_2$ $NO_2$ sein kann;

$R_3$ Halogen bedeutet;

$R_4$ für Wasserstoff oder die Gruppe $-C(O)R'$ steht, wobei R' unsubstituiertes oder durch Halogen, $C_1$–$C_3$-Alkoxy oder $C_1$–$C_3$-Alkylthio substituiertes $C_1$–$C_4$-Alkyl bedeutet;

$R_5$, $R_6$ und $R_7$ unabhängig voneinander für Wasserstoff, Halogen, Nitro, Cyano, Rhodano, $C_1$–$C_{12}$-Alkyl, $C_3$–$C_8$-Cycloalkyl, $C_1$–$C_8$-Alkylthio, $C_1$–$C_6$-Alkylsulfonyl, $C_1$–$C_6$-Alkylsulfoxyl, $C_3$–$C_6$-Alkenyl, $C_3$–$C_6$-Haloalkenyl, $C_3$–$C_6$-Alkinyl, $C_3$–$C_6$-Haloalkinyl, $C_3$–$C_6$-Alkenyloxy, $C_3$–$C_6$-Haloalkenyloxy,

$C_3$–$C_6$-Alkenylthio, $C_3$–$C_6$-Alkinyloxy, $C_3$–$C_6$-Haloalkinyloxy, durch Halogen, Cyano, und/oder $C_1$–$C_4$-Alkoxy substituiertes $C_1$–$C_8$-Alkyl, unsubstituiertes oder durch Halogen substituiertes $C_1$–$C_8$-Alkoxy, dessen Alkylteil gegebenenfalls durch ein oder mehrere einzelne Sauerstoffatome unterbrochen ist oder für die Gruppe $Q–(E)_n–(X)_m$ stehen, wobei n für 0 oder 1 steht, m für 0 oder 1 steht, Q für ein unsubstituiertes oder durch Halogen, Nitro, $C_1$–$C_3$-Alkyl, $CF_3$ und/oder $C_1$–$C_3$-Alkoxy substituiertes Phenyl oder einen gesättigten oder ungesättigten fünf- oder sechsgliedrigen Heterocyclus mit einem bis drei Heteroatomen, wie Sauerstoff, Stickstoff oder Schwefel, steht, E eine $C_1$–$C_3$ Alkylenbrücke bedeutet und X für Sauerstoff oder Schwefel steht.

3. Verfahren nach Anspruch 2, zur Herstellung von Verbindungen worin $R_1$, $R_2$, $R_3$ und $R_4$ die unter Formel I angegebenen Bedeutungen haben und $R_5$, $R_6$ und $R_7$ unabhängig voneinander für Wasserstoff, Halogen, Nitro, Cyano, $C_1$–$C_6$-Alkyl, Cyclopentyl, Cyclohexyl, $C_1$–$C_3$-Alkylthio, $C_1$–$C_3$-Alkylsulfonyl, $C_1$–$C_3$-Alkylsulfoxyl, $C_3$–$C_4$-Alkenyl, Propargyl, durch Halogen, Cyano und/oder $C_1$–$C_3$-Alkoxy substituiertes $C_1$–$C_3$-Alkyl, $C_1$–$C_6$-Alkoxy, dessen Alkylteil durch ein bis 2 einzelne Sauerstoffatome unterbrochen ist oder für die Gruppe $Q–(E)_n–(X)_m$ stehen, worin n für 0 oder 1 und m für 0 oder 1 steht, Q für ein unsubstituiertes oder durch Halogen, Nitro, Methyl, $CF_3$ oder Methoxy substituiertes Phenyl oder eine Pyridylgruppe steht, E für eine Methylenbrücke steht und X Sauerstoff oder Schwefel bedeutet.

4. Verfahren nach Anspruch 3, zur Herstellung von Verbindungen der Formel I, worin $R_1$, $R_2$ und $R_3$ die unter Formel I angegebenen Bedeutungen haben, $R_4$ für Wasserstoff steht, und $R_5$, $R_6$ und $R_7$ unabhängig voneinander für Wasserstoff, Halogen, Nitro, Cyano, $C_1$–$C_6$-Alkyl, Cyclopentyl, Cyclohexyl, $C_1$–$C_3$-Alkylthio, $C_1$–$C_3$-Alkylsulfonyl, $C_1$–$C_3$-Alkylsulfoxyl, $C_3$–$C_4$-Alkenyl, Propargyl durch Halogen, Cyano und/oder $C_1$–$C_3$-Alkoxy substituiertes $C_1$–$C_3$-Alkyl, $C_1$–$C_6$-Alkoxy, dessen Alkylteil durch ein bis 2 einzelne Sauerstoffatome unterbrochen ist oder für die Gruppe $Q–(E)_n–(X)_m$ stehen, worin n für 0 oder 1 und m für 0 oder 1 steht, Q für ein unsubstituiertes oder durch Halogen, Nitro, Methyl, $CF_3$ oder Methoxy substituiertes Phenyl oder eine Pyridylgruppe steht, E für eine Methylenbrücke steht und X Sauerstoff oder Schwefel bedeutet.

5. Verfahren nach Anspruch 4, zur Herstellung von Verbindungen der Formel I, worin $R_1$ für $NO_2$ oder $CF_3$ steht; mit der Massgabe, dass nur $R_1$ oder $R_2$ $NO_2$ sein kann; $R_2$ für $NO_2$ oder $CF_3$ steht; $R_3$ Chlor bedeutet; $R_4$ für Wasserstoff steht; $R_6$ Wasserstoff bedeutet und $R_5$ und $R_7$ unabhängig voneinander für Wasserstoff, Fluor, Chlor, Brom, $C_1$–$C_3$-Alkyl, $C_1$–$C_3$-Haloalkyl, $C_1$–$C_3$-Alkoxy, $C_1$–$C_3$-Haloalkoxy, unsubstituiertes oder durch Halogen substituiertes Phenoxy, $OCH_2OCH_3$, $OC_2H_4OCH_3$, $OCH_2OC_2H_5$, $OC_2H_4OC_2H_5$, $OC_2H_4OC_2H_4OC_2H_5$ oder –S–(2-Pyridyl) stehen.

6. Verfahren nach Anspruch 1, zur Herstellung einer Verbindung der Formel I, ausgewählt aus der Reihe:
N-(3′-Chlor-2′,6′-dinitro-4′-trifluormethylphenyl)-2-amino-4,6-dichlorpyrimidin,
N-(3′-Chlor-2′,6′-dinitro-4′-trifluormethylphenyl)-2-amino-4-chlor-6-trichlormethylpyrimidin,
N-(3′-Chlor-2′,6′-dinitro-4′-trifluormethylphenyl)-2-amino-4,6-dimethylpyrimidin,
N-(3′-Chlor-2′,6′-dinitro-4′-trifluormethylphenyl)-2-amino-4-trichlormethyl-6-(methoxyethoxy)pyrimidin,
N-(3′-Chlor-2′,6′-dinitro-4′-trifluormethylphenyl)-2-amino-4-methoxy-6-(2-pyridylthio)-pyrimidin,
N-(3′-Chlor-2′,6′-dinitro-4′-trifluormethylphenyl)-2-amino-4-trichlormethyl-6-(ethoxyethoxyethoxy)pyrimidin und
N-(3′-Chlor-2′,6′-dinitro-4′-trifluormethylphenyl)-2-amino-4-methoxy-6-(4″-chlorphenoxy)-pyrimidin.

7. Mittel zur Bekämpfung oder Verhütung eines Befalls durch Schädlinge, dadurch gekennzeichnet, dass es als mindestens eine aktive Komponente eine Verbindung der Formel I zusammen mit üblichen Zuschlagstoffen und Trägermaterialien enthält.

8. Mittel gemäss Anspruch 7, dadurch gekennzeichnet, dass es als mindestens eine aktive Komponente eine Verbindung der Formel I gemäss Anspruch 2 enthält.

9. Mittel zur Bekämpfung von Mikroorganismen gemäss Anspruch 7, dadurch gekennzeichnet, dass es als mindestens eine aktive Komponente eine Verbindung der Formel I gemäss einem der Ansprüche 3 bis 5 enthält.

10. Mittel nach Anspruch 7, dadurch gekennzeichnet, dass es 0,1 bis 99% eines Wirkstoffs der Formel I, 99,9 bis 1% eines festen oder flüssigen Zusatzstoffes und 0 bis 25% eines Tensides enthält.

11. Mittel nach Anspruch 7, dadurch gekennzeichnet, dass es 0,1 bis 95% eines Wirkstoffs der Formel I, 99,8 bis 5% eines festen oder flüssigen Zusatzstoffes und 0,1 bis 25% eines Tensids enthält.

12. Verfahren zur Herstellung eines wie in Anspruch 7 beanspruchten agrochemischen Mittels, dadurch gekennzeichnet, dass man mindestens eine gemäss Anspruch 1 definierte Verbindung der Formel I mit geeigneten festen oder flüssigen Zusatzstoffen und Tensiden innig vermischt.

13. Verfahren zur Bekämpfung oder Verhütung eines Befalls von Kulturpflanzen durch phytopathogene Schädlinge, dadurch gekennzeichnet, dass man eine Verbindung der Formel I auf die Pflanze oder deren Standort appliziert.

**Claims for the Contracting States GB, BE, DE, FR, IT, LU, NL, SE, CH, LI**

1. Compounds of the general formula I

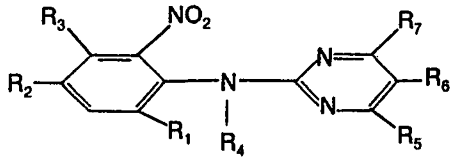 (I)

wherein

$R_1$ and $R_2$ are hydrogen, $NO_2$ or $CF_3$, with the proviso that only $R_1$ or $R_2$ can be $NO_2$,

$R_3$ is halogen,

$R_4$ is hydrogen or the -C(O)R′ group, in which R′ is $C_1$–$C_4$alkyl which is unsubstituted or substituted by halogen, $C_1$–$C_3$alkoxy or $C_1$–$C_3$alkylthio,

$R_5$, $R_6$ and $R_7$ are each independently hydrogen, halogen, nitro, cyano, thiocyano, $C_1$–$C_{12}$alkyl, $C_3$–$C_8$cycloalkyl, $C_1$–$C_8$alkylthio, $C_1$–$C_6$alkylsulfonyl, $C_1$–$C_6$alkylsulfoxyl, $C_3$–$C_6$alkenyl, $C_3$–$C_6$haloalkenyl, $C_3$–$C_6$alkynyl, $C_3$–$C_6$haloalkynyl, $C_3$–$C_6$alkenyloxy, $C_3$–$C_6$haloalkenyloxy, $C_3$–$C_6$alkenylthio, $C_3$–$C_6$alkynyloxy, $C_3$–$C_6$haloalkynyloxy, $C_1$–$C_8$alkyl which is substituted by halogen, cyano and/or $C_1$–$C_4$alkoxy, unsubstituted $C_1$–$C_8$alkoxy or halogen-substituted $C_1$–$C_8$alkoxy, the alkyl moiety of which may be interrupted by one or more single oxygen atoms; or are the Q–$(E)_n$–$(X)_m$– group, in which n is 0 or 1, m is 0 or 1, Q is phenyl which is unsubstituted or substituted by halogen, nitro, $C_1$–$C_3$alkyl, $CF_3$ and/or $C_1$–$C_3$alkoxy, or is a saturated or unsaturated 5- or 6-membered heterocyclic radical containing one to three identical or different hetero atoms, such as oxygen, nitrogen or sulfur, E is a $C_1$–$C_3$alkylene bridge, and X is oxygen or sulfur.

2. Compounds of the formula I according to claim 1, wherein

$R_1$ is $NO_2$ or $CF_3$,

$R_2$ is $NO_2$ or $CF_3$, with the proviso that only $R_1$ or $R_2$ can be $NO_2$,

$R_3$ is halogen,

$R_4$ is hydrogen or the -C(O)R′ group, in which R′ is $C_1$–$C_4$alkyl which is unsubstituted or substituted by halogen, $C_1$–$C_3$alkoxy or $C_1$–$C_3$alkylthio,

$R_5$, $R_6$ and $R_7$ are each independently hydrogen, halogen, nitro, cyano, thiocyano, $C_1$–$C_{12}$alkyl, $C_3$–$C_8$cycloalkyl, $C_1$–$C_8$alkylthio, $C_1$–$C_6$alkylsulfonyl, $C_1$–$C_6$alkylsulfoxyl, $C_3$–$C_6$alkenyl, $C_3$–$C_6$haloalkenyl, $C_3$–$C_6$alkynyl, $C_3$–$C_6$haloalkynyl, $C_1$–$C_8$alkyl which is substituted by halogen, cyano and/or $C_1$–$C_4$alkoxy, unsubstituted $C_1$–$C_8$alkoxy or halogen-substituted $C_1$–$C_8$alkoxy, the alkyl moiety of which may be interrupted by one or more single oxygen atoms; or are the Q–$(E)_n$–$(X)_m$– group, in which n is 0 or 1, m is 0 or 1, Q is phenyl which is unsubstituted or substituted by halogen, nitro, $C_1$–$C_3$alkyl, $CF_3$ and/or $C_1$–$C_3$alkoxy, or is a saturated or unsaturated 5- or 6-membered heterocyclic radical containing one to three hetero atoms, such as oxygen, nitrogen or sulfur, E is a $C_1$–$C_3$alkylene bridge, and X is oxygen or sulfur.

3. Compounds of the formula I according to claim 2, wherein $R_1$, $R_2$, $R_3$ and $R_4$ are as defined for formula I and $R_5$, $R_6$ and $R_7$ are each independently hydrogen, halogen, nitro, cyano, $C_1$–$C_6$alkyl, cyclopentyl, cyclohexyl, $C_1$–$C_3$alkylthio, $C_1$–$C_3$alkylsulfonyl, $C_1$–$C_3$alkylsulfoxyl, $C_3$–$C_4$alkenyl, propargyl, $C_1$–$C_3$alkyl which is substituted by halogen, cyano and/or $C_1$–$C_3$alkoxy, or are $C_1$–$C_6$alkoxy, the alkyl moiety of which is interrupted by 1 or 2 single oxygen atoms, or are the Q–$(E)_n$–$(X)_m$– group, in which n is 0 or 1, m is 0 or 1, Q is phenyl which is unsubstituted or substituted by halogen, nitro, methyl, $CF_3$ or methoxy, or is a pyridyl group, E is a methylene bridge, and X is oxygen or sulfur.

4. Compounds of the formula I according to claim 3, wherein $R_1$, $R_2$ and $R_3$ are as defined for formula I, $R_4$ is hydrogen and $R_5$, $R_6$ and $R_7$ are each independently hydrogen, halogen, nitro, cyano, $C_1$–$C_6$alkyl, cyclopentyl, cyclohexyl, $C_1$–$C_3$alkylthio, $C_1$–$C_3$alkylsulfonyl, $C_1$–$C_3$alkylsulfoxyl, $C_3$–$C_4$alkenyl, propargyl, $C_1$–$C_3$alkyl, which is substituted by halogen, cyano and/or $C_1$–$C_3$alkoxy, $C_1$–$C_6$alkoxy, the alkyl moiety of which is interrupted by 1 or 2 single oxygen atoms, or are the Q–$(E)_n$–$(X)_m$– group, in which n is 0 or 1, m is 0 or 1, Q is phenyl which is unsubstituted or substituted by halogen, nitro, methyl, $CF_3$ or methoxy, or is a pyridyl group, E is a methylene bridge, and X is oxygen or sulfur.

5. Compounds of the formula I according to claim 4, wherein $R_1$ is $NO_2$ or $CF_3$, with the proviso that only $R_1$ or $R_2$ can be $NO_2$, $R_2$ is $NO_2$ or $CF_3$, $R_3$ is chlorine, $R_4$ is hydrogen, $R_6$ is hydrogen and $R_5$ and $R_7$ are each independently hydrogen, fluorine, chlorine, bromine, $C_1$–$C_3$alkyl, $C_1$–$C_3$haloalkyl, $C_1$–$C_3$alkoxy, $C_1$–$C_3$haloalkoxy, unsubstituted or halogen-substituted phenoxy, $OCH_2OCH_3$, $OC_2H_4OCH_3$, $OCH_2OC_2H_5$, $OC_2H_4OC_2H_5$, $OC_2H_4OC_2H_4OC_2H_5$ or –S–(2-pyridyl).

6. A compound of the formula I according to claim 1, selected from the group consisting of:

N-(3′-chloro-2′,6′-dinitro-4′-trifluoromethylphenyl)-2-amino-4,6-dichloropyrimidine,

N-(3′-chloro-2′,6′-dinitro-4′-trifluoromethylphenyl)-2-amino-4-chloro-6-trichloromethylpyrimidine,

N-(3′-chloro-2′,6′-dinitro-4′-trifluoromethylphenyl)-2-amino-4,6-dimethylpyrimidine,

N-(3′-chloro-2′,6′-dinitro-4′-trifluoromethylphenyl)-2-amino-4-trichloromethyl-6-(methoxyethoxy)pyrimidine,

N-(3′-chloro-2′,6′-dinitro-4′-trifluoromethylphenyl)-2-amino-4-methoxy-6-(2-pyridylthio)-pyrimidine,

N-(3′-chloro-2′,6′-dinitro-4′-trifluoromethylphenyl)-2-amino-4-trichloromethyl-6-(ethoxyethoxyethoxy)pyrimidine, and

N-(3′-chloro-2′,6′-dinitro-4′-trifluoromethylphenyl)-2-amino-4-methoxy-6-(4″-chlorophenoxy)pyrimidine.

7. A process for the preparation of compounds of the formula I, which comprises reacting a compound of the formula II

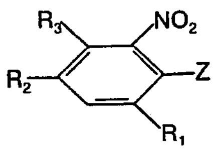

(II)

with a pyrimidine derivative of the formula III

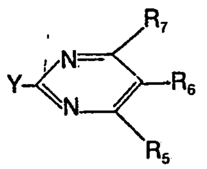

(III)

in the presence of a base, to give a compound of the formula I′

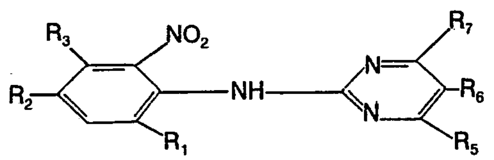

(I′)

and, to obtain N-acylated derivatives, N-acylating the compound of the formula I′ with a reactive derivative of the carboxylic acid IV

$R_4COOH$ (IV)

in which formulae the substituents $R_1$ to $R_7$ are as defined for formula I and Z and Y are $NH_2$ or halogen, with the proviso that, if Z is halogen, Y is $NH_2$ and, if Z is $NH_2$, Y is halogen.

8. A composition for controlling or preventing attack by phytopathogenic pests, which contains, as at least one active component, a compound of the formula I, together with conventional adjuvants and carriers.

9. A composition according to claim 8, which contains, as at least one active component, a compound of the formula I according to claim 2.

10. A composition for controlling microorganisms according to claim 8, which contains, as at least one active component, a compound of the formula I according to any one of claims 3 to 6.

11. A method of controlling or preventing attacks on cultivated plants by phytopathogenic pests, which comprises applying to said plants or to the locus thereof a compound of the formula I.

**Claims for the Contracting State AT**

1. A process for the preparation of compounds of the formula I

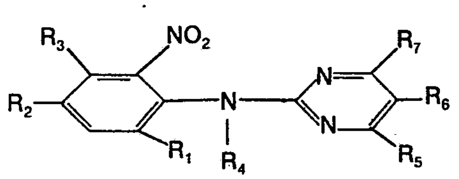

(I)

wherein

$R_1$ and $R_2$ are hydrogen, $NO_2$ or $CF_3$, with the proviso that only $R_1$ or $R_2$ can be $NO_2$,

$R_3$ is halogen,

$R_4$ is hydrogen or the $-C(O)R'$ group, in which R′ is $C_1$–$C_4$alkyl which is unsubstituted or substituted by halogen, $C_1$–$C_3$alkoxy or $C_1$–$C_3$alkylthio,

$R_5$, $R_6$ and $R_7$ are each independently hydrogen, halogen, nitro, cyano, thiocyano, $C_1$–$C_{12}$alkyl, $C_3$–$C_8$cycloalkyl, $C_1$–$C_8$alkylthio, $C_1$–$C_6$alkylsulfonyl, $C_1$–$C_6$alkylsulfoxyl, $C_3$–$C_6$alkenyl, $C_3$–$C_6$haloalkenyl, $C_3$–$C_6$alkynyl, $C_3$–$C_6$haloalkynyl, $C_3$–$C_6$alkenyleneoxy, $C_3$–$C_6$haloalkenyloxy, $C_3$–$C_6$alkenylthio, $C_3$–$C_6$alkynyloxy, $C_3$–$C_6$haloalkynyloxy, $C_1$–$C_8$alkyl
which is substituted by halogen, cyano and/or $C_1$–$C_4$alkoxy, unsubstituted $C_1$–$C_8$alkoxy or halogen-substituted $C_1$–$C_8$alkoxy, the alkyl moiety of which may be interrupted by one or more single oxygen atoms; or are the $Q-(E)_n-(X)_m-$ group, in which n is 0 or 1, m is 0 or 1, Q is phenyl which is unsubstituted or substituted by halogen, nitro, $C_1$–$C_3$alkyl, $CF_3$ and/or $C_1$–$C_3$alkoxy, or is a saturated or unsaturated 5- or 6-membered heterocyclic radical containing one to three identical or different hetero atoms, such as oxygen, nitrogen or sulfur, E is a $C_1$–$C_3$alkylene bridge, and X is oxygen or sulfur,
which comprises reacting a compound of the formula II

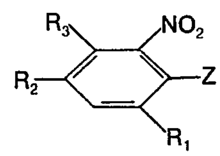

(II)

with a pyrimidine derivative of the formula III

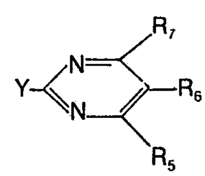

(III)

in the presence of a base, to give a compound of the formula I′

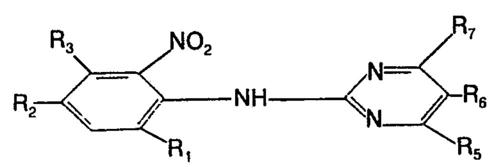

(I′)

and, to obtain N-acylated derivatives, N-acylating the compound of the formula I′ with a reactive derivative of the carboxylic acid IV

$R_4COOH$ (IV)

in which formulae the substituents $R_1$ to $R_7$ are as defined for formula I and Z and Y are $NH_2$ or halogen, with the proviso that, if Z is halogen, Y is $NH_2$ and, if Z is $NH_2$, Y is halogen.

2. A process according to claim 1 for the preparation of compounds of the formula I wherein

$R_1$ is $NO_2$ or $CF_3$,

$R_2$ is $NO_2$ or $CF_3$, with the proviso that only $R_1$ or $R_2$ can be $NO_2$,

$R_3$ is halogen,

$R_4$ is hydrogen or the –C(O)R′ group, in which R′ is $C_1$–$C_4$alkyl which is unsubstituted or substituted by halogen, $C_1$–$C_3$alkoxy or $C_1$–$C_3$alkylthio,

$R_5$, $R_6$ and $R_7$ are each independently hydrogen, halogen, nitro, cyano, thiocyano, $C_1$–$C_{12}$alkyl, $C_3$–$C_8$cycloalkyl, $C_1$–$C_8$alkylthio, $C_1$–$C_6$alkylsulfonyl, $C_1$–$C_6$alkylsulfoxyl, $C_3$–$C_6$alkenyl, $C_3$–$C_6$haloalkenyl, $C_3$–$C_6$alkynyl, $C_3$–$C_6$haloalkynyl, $C_3$–$C_6$alkenyloxy, $C_3$–$C_6$haloalkenyloxy, $C_3$–$C_6$alkenylthio, $C_3$–$C_6$alkynyloxy, $C_3$–$C_6$haloalkynyloxy, $C_1$–$C_8$alkyl which is substituted by halogen, cyano and/or $C_1$–$C_4$alkoxy, unsubstituted $C_1$–$C_8$alkoxy or halogen-substituted $C_1$–$C_8$alkoxy, the alkyl moiety of which may be interrupted by one or more single oxygen atoms; or are the Q–$(E)_n$–$(X)_m$– group, in which n is 0 or 1, m is 0 or 1, Q is phenyl which is unsubstituted or substituted by halogen, nitro, $C_1$–$C_3$alkyl, $CF_3$ and/or $C_1$–$C_3$alkoxy, or is a saturated or unsaturated 5- or 6-membered heterocyclic radical containing one to three hetero atoms, such as oxygen, nitrogen or sulfur, E is a $C_1$–$C_3$alkylene bridge, and X is oxygen or sulfur.

3. A process according to claim 2 for the preparation of compounds wherein $R_1$, $R_2$, $R_3$ and $R_4$ are as defined for formula I and $R_5$, $R_6$ and $R_7$ are each independently hydrogen, halogen, nitro, cyano, $C_1$–$C_6$alkyl, cyclopentyl, cyclohexyl, $C_1$–$C_3$alkylthio, $C_1$–$C_3$alkylsulfonyl, $C_1$–$C_3$alkylsulfoxyl, $C_3$–$C_4$alkenyl, propargyl, $C_1$–$C_3$alkyl which is substituted by halogen, cyano and/or $C_1$–$C_3$alkoxy, or are $C_1$–$C_6$alkoxy, the alkyl moiety of which is interrupted by 1 or 2 single oxygen atoms, or are the Q–$(E)_n$–$(X)_m$–U group, in which n is 0 or 1, m is 0 or 1, Q is phenyl which is unsubstituted or substituted by halogen, nitro, methyl, $CF_3$ or methoxy, or is a pyridyl group, E is a methylene bridge, and X is oxygen or sulfur.

4. A process according to claim 3 for the preparation of compounds of the formula I wherein $R_1$, $R_2$ and $R_3$ are as defined for formula I, $R_4$ is hydrogen and $R_5$, $R_6$ and $R_7$ are each independently hydrogen, halogen, nitro, cyano, $C_1$–$C_6$alkyl, cyclopentyl, cyclohexyl, $C_1$–$C_3$alkylthio, $C_1$–$C_3$alkylsulfonyl, $C_1$–$C_3$alkylsulfoxyl, $C_3$–$C_4$alkenyl, propargyl, $C_1$–$C_3$alkyl which is substituted by halogen, cyano and/or $C_1$–$C_3$alkoxy, $C_1$–$C_6$alkoxy, the alkyl moiety of which is interrupted by 1 or 2 single oxygen atoms, or are the Q–$(E)_n$–$(X)_m$– group, in which n is 0 or 1, m is 0 or 1, Q is phenyl which is unsubstituted or substituted by halogen, nitro, methyl, $CF_3$ or methoxy, or is a pyridyl group, E is a methylene bridge, and X is oxygen or sulfur.

5. A process according to claim 4 for the preparation of compounds of the formula I wherein $R_1$ is $NO_2$ or $CF_3$, with the proviso that only $R_1$ or $R_2$ can be $NO_2$, $R_2$ is $NO_2$ or $CF_3$, $R_3$ is chlorine, $R_4$ is hydrogen, $R_6$ is hydrogen and $R_5$ and $R_7$ are each independently hydrogen, fluorine, chlorine, bromine, $C_1$–$C_3$alkyl, $C_1$–$C_3$haloalkyl, $C_1$–$C_3$alkoxy, $C_1$–$C_3$haloalkoxy, unsubstituted or halogen-substituted phenoxy, $OCH_2OCH_3$, $OC_2H_4OCH_3$, $OCH_2OC_2H_5$, $OC_2H_4OC_2H_5$, $OC_2H_4OC_2H_4OC_2H_5$ or –S–(2-pyridyl).

6. A process according to claim 1 for the preparation of a compound of the formula I selected from the group consisting of:

N-(3′-chloro-2′,6′-dinitro-4′-trifluoromethylphenyl)-2-amino-4,6-dichloropyrimidine,

N-(3′-chloro-2′,6′-dinitro-4′-trifluoromethylphenyl)-2-amino-4-chloro-6-trichloromethylpyrimidine,

N-(3′-chloro-2′,6′-dinitro-4′-trifluoromethylphenyl)-2-amino-4,6-dimethylpyrimidine,

N-(3′-chloro-2′,6′-dinitro-4′-trifluoromethylphenyl)-2-amino-4-trichloromethyl-6-(methoxyethoxy)pyrimidine,

N-(3′-chloro-2′,6′-dinitro-4′-trifluoromethylphenyl)-2-amino-4-methoxy-6-(2-pyridylthio)-pyrimidine,

N-(3′-chloro-2′,6′-dinitro-4′-trifluoromethylphenyl)-2-amino-4-trichloromethyl-6-(ethoxyethoxyethoxy)pyrimidine, and

N-(3′-chloro-2′,6′-dinitro-4′-trifluoromethylphenyl)-2-amino-4-methoxy-6-(4″-chlorophenoxy)pyrimidine.

7. A composition for controlling or preventing attack by pests, which contains, as at least one active component, a compound of the formula I, together with conventional adjuvants and carriers.

8. A composition according to claim 7, which contains, as at least one active component, a compound of the formula I according to claim 2.

9. A composition for controlling microorganisms according to claim 7, which contains, as at least one active component, a compound of the formula I according to any one of claims 3 to 5.

10. A composition according to claim 7, which contains 0.1 to 99% of a compound of the formula I, 99.9 to 1% of a solid or liquid adjuvant, and 0 to 25% of a surfactant.

11. A composition according to claim 7, which contains 0.1 to 95% of a compound of the formula I, 99.8 to 5% of a solid or liquid adjuvant, and 0.1 to 25% of a surfactant.

12. A process for the preparation of an agrochemical composition as claimed in claim 7, which comprises intimately mixing at least one compound of the formula I as defined in claim 1 with suitable solid or liquid adjuvants and surfactants.

13. A method of controlling or preventing attacks on cultivated plants by phytopathogenic pests, which comprises applying to said plants or to the locus thereof a compound of the formula I.

**Revendications pour les Etats contractants: GB, BE, DE, FR, IT, LU, NL, SE, CH, LI**

1. Composés de formule générale I

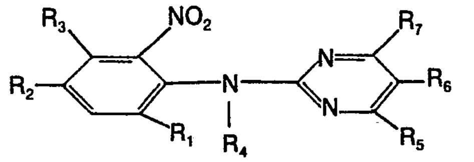 (I)

dans laquelle

$R_1$ et $R_2$ représentent l'hydrogène, un groupe $NO_2$ ou $CF_3$, sous réserve qu'un seul de ces symboles $R_1$ ou $R_2$ peut représenter $NO_2$;

$R_3$ représente un halogène;

$R_4$ représente l'hydrogène ou le groupe –C(O)R' dans lequel R' représente un groupe alkyle en C1–C4 non substitué ou substitué pas des halogènes, des groupes alcoxy en C1–C3 ou alkylthio en C1–C3;

$R_5$, $R_6$ et $R_7$ représentent chacun, indépendamment les uns des autres, l'hydrogène, un halogène, un groupe nitro, cyano, sulfocyano, alkyle en C1–C12, cycloalkyle en C3–C8, alkylthio en C1–C8, alkylsulfonyle en C1–C6, alkylsulfoxyle en C1–C6, alcényle en C3–C6, halogénoalcényle en C3–C6, alcynyle en C3–C6, halogénoalcynyle en C3–C6, alcényloxy en C3–C6, halogénoalcényloxy en C3–C6, alcénylthio en C3–C6, alcynyloxy en C3–C6, halogénoalcynyloxy en C3–C6, un groupe alkyle en C1–C8 substitué par des halogènes, des groupes cyano et/ou alcoxy en C1–C4, un groupe alcoxy en C1–C8 non substitué ou substitué par des halogènes et dont la partie alkyle est éventuellement interrompue par un ou plusieurs atomes d'oxygène isolés, ou le groupe $Q–(E)_n–(X)_m$ dans lequel n est égal à 0 ou 1, m est égal à 0 ou 1, Q représente un groupe phényle non substitué ou substitué pas des halogènes, des groupes nitro, alkyle en C1–C3, $CF_3$ et/ou alcoxy en C1–C3, ou un hétérocycle saturé ou insaturé à 5 ou 6 chaînons contenant 1 à 3 hétéroatomes identiques ou différents tels que l'oxygène, l'azote ou le soufre, E représente un pont alkylène en C1–C3 et X représente l'oxygène ou le soufre.

2. Composés de formule I selon la revendication 1, caractérisés en ce que

$R_1$ représente $NO_2$ ou $CF_3$;

$R_2$ représente $NO_2$ ou $CF_3$; sous réserve qu'un seul des symboles $R_1$ et $R_2$ peut représenter $NO_2$;

$R_3$ représente un halogène;

$R_4$ représente l'hydrogène ou le groupe –C(O)R' dans lequel R' représente un groupe alkyle en C1–C4 non substitué ou substitué pas des halogènes, des groupes alcoxy en C1–C3 ou alkylthio en C1–C3;

$R_5$, $R_6$ et $R_7$ représentent chacun, indépendamment les uns des autres, l'hydrogène, un halogène, un groupe nitro, cyano, sulfocyano, alkyle en C1–C12, cycloalkyle en C3–C8, alkylthio en C1–C8, alkylsulfonyle en C1–C6, alkylsulfoxyle en C1–C6, alcényle en C3–C6, halogénoalcényle en C3–C6, alcynyle en C3–C6, halogénoalcynyle en C3–C6, alkyle en C1–C8 substitué pas des halogènes, des groupes cyano et/ou alcoxy en C1–C4, alcoxy en C1–C8 non substitué ou substitué pas des halogènes et dont la partie alkyle est éventuellement interrompue par un ou plusieurs atomes d'oxygène isolés, ou le groupe $Q–(E)_n–(X)_m$ dans lequel n est égal à 0 ou 1, m est égal à 0 ou 1, Q représente un groupe phényle non substitué ou substitué pas des halogènes, des groupes nitro, alkyle en C1–C3, $CF_3$ et/ou alcoxy en C1–C3, ou un hétérocycle saturé ou insaturé à 5 ou 6 chaînons contenant 1 à 3 hétéroatomes tels que l'oxygène, l'azote ou le soufre, E représente un pont alkylène en C1–C3 et X représente l'oxygène ou le soufre.

3. Composés de formule I selon la revendication 2, caractérisés en ce que $R_1$, $R_2$, $R_3$ et $R_4$ ont les significations indiquées en référence à la formule I et $R_5$, $R_6$ et $R_7$ représentent chacun, indépendamment les uns des autres, l'hydrogène, un halogène, un groupe nitro, cyano, alkyle en C1–C6, cyclopentyle, cyclohexyle, alkylthio en C1–C3, alkylsulfonyle en C1–C3, alkylsulfoxyle en C1–C3, alcényle en C3–C4, propargyle, alkyle en C1–C3 substitué par des halogènes, des groupes cyano et/ou alcoxy en C1–C3, alcoxy en C1–C6 dont la partie alkyle est interrompue par 1 à 2 atomes d'oxygène isolés, ou le groupe $Q–(E)_n–(X)_m$ dans lequel n est égal à 0 ou 1, m est égal à 0 ou 1, Q représente un groupe phényle non substitué ou substitué pas des halogènes, des groupes nitro, méthyle, $CF_3$ ou méthoxy, ou un groupe pyridyle, E représente un pont méthylène et X représente l'oxygène ou le soufre.

4. Composés de formule I selon la revendication 3, caractérisés en ce que $R_1$, $R_2$ et $R_3$ ont les significations indiquées en référence à la formule I, $R_4$ représente l'hydrogène et $R_5$, $R_6$ et $R_7$ représentent chacun, indépendamment les uns des autres, l'hydrogène, un halogène, un groupe nitro, cyano, alkyle en C1–C6, cyclopentyle, cyclohexyle, alkylthio en C1–C3, alkylsulfonyle en C1–C3, alkylsulfoxyle en C1–C3, alcényle en C3–C4, propargyle, alkyle en C1–C3 substitué par des halogènes, des groupes cyano et/ou alcoxy en C1–C3, alcoxy en C1–C6 dont la partie alkyle est interrompue par 1 à 2 atomes d'oxygène isolés, ou le groupe $Q–(E)_n–(X)_m$ dans lequel n est égal à 0 ou 1 et m est égal à 0 ou 1, Q représente un groupe phényle non substitué ou substitué pas des halogènes, des groupes nitro, méthyle, $CF_3$ ou méthoxy ou un groupe pyridyle, E représente un pont méthylène et X l'oxygène ou le soufre.

5. Composés de formule I selon la revendication 4, caractérisés en ce que $R_1$ représente $NO_2$ ou $CF_3$; sous réserve qu'un seul des symboles $R_1$ et $R_2$ peut représenter $NO_2$; $R_2$ représente $NO_2$ ou $CF_3$; $R_3$ représente le chlore; $R_4$ représente l'hydrogène; $R_6$ représente l'hydrogène et $R_5$ et $R_7$ représentent chacun, indépendamment l'un de l'autre, l'hydrogène, le fluor, le chlore, le brome, un groupe alkyle en C1–C3, halogénoalkyle en C1–C3, alcoxy en C1–C3, halogénoalcoxy en C1–C3, phénoxy non substitué ou substitué par des halogènes, $OCH_2OCH_3$, $OC_2H_4OCH_3$, $OCH_2OC_2H_5$, $OC_2H_4OC_2H_5$, $OC_2H_4OC_2H_4OC_2H_5$, ou –S–(2-pyridyle).

6. Un composé de formule I selon la revendication 1, choisi dans le groupe suivant:

N-(3'-chloro-2',6'-dinitro-4'-trifluorométhyl-phényl)-2-amino-4,6-dichloropyrimidine,

N-(3′-chloro-2′,6′-dinitro-4′-trifluorométhyl-phényl)-2-amino-4-chloro-6-trichlorométhyl-pyrimidine,
N-(3′-chloro-2′,6′-dinitro-4′-trifluorométhyl-phényl)-2-amino-4,6-diméthylpyrimidine,
N-(3′-chloro-2′,6′-dinitro-4′-trifluorométhyl-phényl)-2-amino-4-trichlorométhyl-6-(méthoxy-éthoxy)-pyrimidine,
N-(3′-chloro-2′,6′-dinitro-4′-trifluorométhyl-phényl)-2-amino-4-méthoxy-6-(2-pyridylthio)-pyrimidine,
N-(3′-chloro-2′,6′-dinitro-4′-trifluorométhyl-phényl)-2-amino-4-trichlorométhyl-6-(éthoxy-éthoxyéthoxy)-pyrimidine, et
N-(3′-chloro-2′,6′-dinitro-4′-trifluorométhyl-phényl)-2-amino-4-méthoxy-6-(4″-chloro-phénoxy)-pyrimidine.

7. Procédé de préparation des composés de formule I, caractérisé en ce que l'on fait réagir un composé de formule II

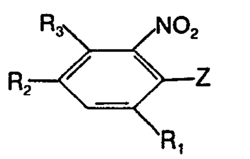

(II)

en présence d'une base, avec un dérivé de la pyrimidine répondant à la formule III

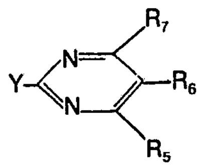

(III)

ce qui donne un composé de formule I′

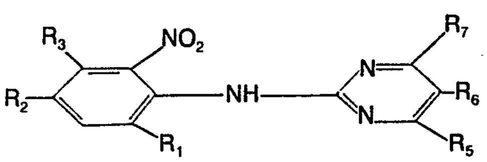

(I′)

qu'on soumet, pour la préparation des dérivés N-acylés, à N-acylation à l'aide d'un dérivé réactif de l'acide carboxylique IV

$$R_4COOH \quad (IV)$$

les symboles $R_1$ à $R_7$ ayant les significations indiquées en référence à la formule I et Z et Y représentent $NH_2$ ou un halogène, sous réserve que, lorsque Z représente un halogène, Y représente $NH_2$, et lorsque Z représente $NH_2$, Y représente un halogène.

8. Produit pour combattre ou prévenir une attaque par des parasites phytopathogènes, caractérisé en ce qu'il contient au moins un composant actif consistant en un composé de formule I, avec des additifs et véhicules usuels.

9. Produit selon la revendication 8, caractérisé en ce qu'il contient au moins un composant actif consistant en un composé de formule I selon la revendication 2.

10. Produit pour combattre les microorganismes selon la revendication 8, caractérisé en ce qu'il contient au moins un composant actif consistant en un composé de formule I selon l'une des revendications 3 à 6.

11. Procédé pour combattre ou prévenir une attaque de végétaux cultivés par des parasites phytopathogènes, caractérisé en ce que l'on applique un composé de formule I sur la plante ou son habitat.

**Revendications pour l'Etat contractant: AT**

1. Procédé de préparation des composés de formule I

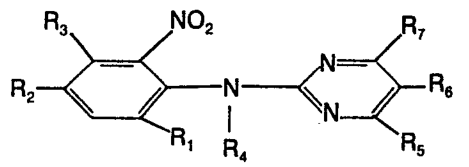

(I)

dans laquelle

$R_1$ et $R_2$ représentent l'hydrogène, un groupe $NO_2$ ou $CF_3$, sous réserve qu'un seul des symboles $R_1$ ou $R_2$ peut représenter $NO_2$;

$R_3$ représente un halogène;

$R_4$ représente l'hydrogène ou le groupe $-C-(O)R'$ dans lequel R′ représente un groupe alkyle en C1–C4 non substitué ou substitué par des halogènes, des groupes alcoxy en C1–C3 ou alkylthio en C1–C3;

$R_5$, $R_6$ et $R_7$ représentent chacun, indépendamment les uns des autres, l'hydrogène, un halogène, un groupe nitro, cyano, sulfocyano, alkyle en C1–C12, cycloalkyle en C3–C8, alkylthio en C1–C8, alkylsulfonyle en C1–C6, alkylsulfoxyle en C1–C6, alcényle en C3–C6, halogénoalcényle en C3–C6, alcynyle en C3–C6, halogénoalcynyle en C3–C6, alcénylène-oxy en C3–C6, halogénoalcényloxy en C3–C6, alcénylthio en C3–C6, alcynyloxy en C3–C6, halogénoalcynyloxy en C3–C6, un groupe alkyle en C1–C8 substitué par des halogènes, des groupes cyano et/ou alcoxy en C1–C4, un groupe alcoxy en C1–C8 non substitué ou substitué par des halogènes et dont la partie alkyle est éventuellement interrompue par un ou plusieurs atomes d'oxygène isolés, ou le groupe $Q-(E)_n-(X)_m-$ dans lequel n est égal à 0 ou 1, m est égal à 0 ou 1, Q représente un groupe phényle non substitué ou substitué par des halogènes, des groupes nitro, alkyle en C1–C3, $CF_3$ et/ou alcoxy en C1–C3 ou un hétérocycle saturé ou insaturé à 5 ou 6 chaînons contenant 1 à 3 hétéroatomes identiques ou différents tels que l'oxygène, l'azote ou le soufre, E représente un pont alkylène en C1–C3 et X représente l'oxygène ou le soufre, caractérisé

en ce que l'on fait réagir un composé de formule II

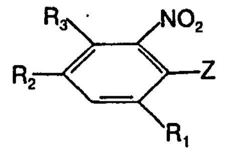

(II)

en présence d'une base, avec un dérivé de la pyrimidine répondant à la formule III

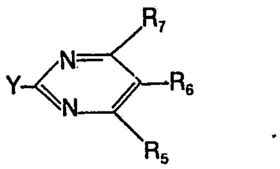

(III)

ce qui donne un composé de formule I'

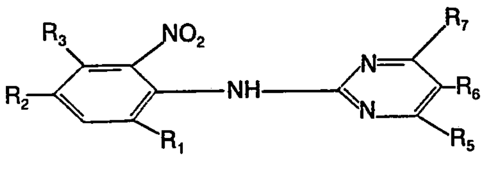

(I')

qu'on soumet, pour préparation des dérivés N-acylés, à N-acylation à l'aide d'un dérivé réactif de l'acide carboxylique IV

$R_4COOH$ (IV)

les symboles $R_1$ à $R_7$ ayant les significations indiquées en référence à la formule I et Z et Y représentant $NH_2$ ou un halogène, sous réserve que lorsque Z représente un halogène, Y représente $NH_2$ et lorsque Z représente $NH_2$, Y représente un halogène.

2. Procédé selon la revendication 1, pour la préparation des composés de formule I dans laquelle

$R_1$ représente $NO_2$ ou $CF_3$;

$R_2$ représente $NO_2$ ou $CF_3$; sous réserve qu'un seul des symboles $R_1$ et $R_2$ peut représenter $NO_2$;

$R_3$ représente un halogène;

$R_4$ représente l'hydrogène ou le groupe $-C(O)R'$ dans lequel R' représente un groupe alkyle en C1–C4 non substitué ou substitué par des halogènes, des groupes alcoxy en C1–C3 ou alkylthio en C1–C3;

$R_5$, $R_6$ et $R_7$ représentent chacun, indépendamment les uns des autres, l'hydrogène, un halogène, un groupe nitro, cyano, sulfocyano, alkyle en C1–C12, cycloalkyle en C3–C8, alkylthio en C1–C8, alkylsulfonyle en C1–C6, alkylsulfoxyle en C1–C6, alcényle en C3–C6, halogénoalcényle en C3–C6, alcynyle en C3–C6, halogénoalcynyle en C3–C6, alcényloxy en C3–C6, halogénoalcényloxy en C3–C6, alcénylthio en C3–C6, alcynyloxy en C3–C6, halogénoalcynyloxy en C3–C6, un groupe alkyle en C1–C8 substitué par des halogènes, des groupes cyano et/ou alcoxy en C1–C4, un groupe alcoxy en C1–C8 non substitué ou substitué pas des halogènes et dont la partie alkyle est éventuellement interrompue par un ou plusieurs atomes d'oxygène isolés, ou le groupe $Q-(E)_n-(X)_m$ dans lequel n est égal à 0 ou 1, m est égal à 0 ou 1, Q représente un groupe phényle non substitué ou substitué par des halogènes, des groupes nitro, alkyle en C1–C3, $CF_3$ et/ou alcoxy en C1–C3, ou un hétérocycle saturé ou insaturé à 5 ou 6 chaînons contenant 1 à 3 hétéroatomes tels que l'oxygène, l'azote ou le soufre, E représente un pont alkylène en C1–C3 et X représente l'oxygène ou le soufre.

3. Procédé selon la revendication 2, pour la préparation de composés dans lesquels $R_1$, $R_2$, $R_3$ et $R_4$ ont les significations indiquées en référence à la formule I et $R_5$, $R_6$ et $R_7$ représentent chacun, indépendamment les uns des autres, l'hydrogène, un halogène, un groupe nitro, cyano, alkyle en C1–C6, cyclopentyle, cyclohexyle, alkylthio en C1–C3, alkylsulfonyle en C1–C3, alkylsulfoxyle en C1–C3, alcényle en C3–C4, propargyle, alkyle en C1–C3 substitué par des halogènes, des groupes cyano et/ou alcoxy en C1–C3, un groupe alcoxy en C1–C6 dont la partie alkyle est interrompue par 1 à 2 atomes d'oxygène isolés, ou le groupe $Q-(E)_n-(X)_m$ dans lequel n est égal à 0 ou 1 et m est égal à 0 ou 1, Q représente un groupe phényle non substitué ou substitué par des halogènes, des groupes nitro, méthyle, $CF_3$ ou méthoxy, ou un groupe pyridyle, E représente un pont méthylène et X l'oxygène ou le soufre.

4. Procédé selon la revendication 3, pour la préparation des composés de formule I dans laquelle $R_1$, $R_2$ et $R_3$ ont les significations indiquées en référence à la formule I, $R_4$ représente l'hydrogène et $R_5$, $R_6$ et $R_7$ représentent chacun, indépendamment les uns des autres, l'hydrogène, un halogène, un groupe nitro, cyano, alkyle en C1–C6, cyclopentyle, cyclohexyle, alkylthio en C1–C3, alkylsulfonyle en C1–C3, alkylsulfoxyle en C1–C3, alcényle en C3–C4, propargyle, alkyle en C1–C3, substitué par des halogènes, des groupes cyano et/ou alcoxy en C1–C3, un groupe alcoxy en C1–C6 dont la partie alkyle est interrompue par 1 à 2 atomes d'oxygène isolés, ou le groupe $Q-(E)_n-(X)_m$ dans lequel n est égal à 0 ou 1 et m est égal à 0 ou 1, Q représente un groupe phényle non substitué ou substitué par des halogènes, des groupes nitro, méthyle, $CF_3$ ou méthoxy, ou un groupe pyridyle, E représente un pont méthylène et X l'oxygène ou le soufre.

5. Procédé selon la revendication 4, pour la préparation de composés de formule I dans laquelle $R_1$ représente $NO_2$ ou $CF_3$; sous réserve qu'un seul des symboles $R_1$ et $R_2$ peut représenter $NO_2$; $R_2$ représente $NO_2$ ou $CF_3$; $R_3$ représente le chlore; $R_4$ représente l'hydrogène; $R_6$ représente l'hydrogène et $R_5$ et $R_7$ représentent chacun, indépendamment l'un de l'autre, l'hydrogène, le fluor, le chlore, le brome, un groupe alkyle en C1–C3, halogénoalkyle en C1–C3, alcoxy en C1–C3, halogénoalcoxy en C1–C3, phénoxy non substitué ou substitué par des halogènes, un groupe $OCH_2OCH_3$, $OC_2H_4QCH_3$, $OCH_2OC_2H_5$, $OC_2H_4OC_2H_5$, $OC_2H_4OC_2H_4OC_2H_5$ ou –S–(2-pyridyle).

6. Procédé selon la revendication 1, pour la préparation d'un composé de formule I choisi dans le groupe suivant:

N-(3'-chloro-2',6'-dinitro-4'-trifluorométhyl-phényl)-2-amino-4,6-dichloropyrimidine,

N-(3'-chloro-2',6'-dinitro-4'-trifluorométhyl-phényl)-2-amino-4-chloro-6-trichlorométhyl-pyrimidine,

N-(3'-chloro-2',6'-dinitro-4'-trifluorométhyl-phényl)-2-amino-4,6-diméthylpyrimidine,

N-(3′-chloro-2′,6′-dinitro-4′-trifluorométhyl-phényl)-2-amino-4-trichlorométhyl-6-(méthoxy-éthoxy)-pyrimidine,
N-(3′-chloro-2′,6′-dinitro-4′-trifluorométhyl-phényl)-2-amino-4-méthoxy-6-(2-pyridylthio)-pyrimidine,
N-(3′-chloro-2′,6′-dinitro-4′-trifluorométhyl-phényl)-2-amino-4-trichlorométhyl-6-(éthoxy-éthoxyéthoxy)-pyrimidine, et
N-(3′-chloro-2′,6′-dinitro-4′-trifluorométhyl-phényl)-2-amino-4-méthoxy-6-(4″-chloro-phénoxy)-pyrimidine.

7. Produit pour combattre ou prévenir une attaque par des parasites, caractérisé en ce qu'il contient au moins un composant actif consistant en un composé de formule I avec des additifs et véhicules usuels.

8. Produit selon la revendication 7, caractérisé en ce qu'il contient au moins un composant actif consistant en un composé de formule I selon la revendication 2.

9. Produit pour combattre les microorganismes selon la revendication 7, caractérisé en ce qu'il contient au moins un composant actif consistant en un composé de formule I selon l'une des revendications 3 à 5.

10. Produit selon la revendication 7, caractérisé en ce qu'il contient de 0,1 à 99% d'une substance active de formule I, de 99,9 à 1% d'un additif solide ou liquide et de 0 à 25% d'un agent tensioactif.

11. Produit selon la revendication 7, caractérisé en ce qu'il contient de 0,1 à 95% d'une substance active de formule I, de 99,8 à 5% d'un additif solide ou liquide et de 0,1 à 25% d'un agent tensioactif.

12. Procédé de préparation d'un produit agrochimique tel que revendiqué dans la revendication 7, caractérisé en ce que l'on mélange intimement au moins un composé de formule I selon la revendication 1 avec des additifs solides ou liquides appropriés et des agents tensioactifs.

13. Procédé pour combattre ou prévenir une attaque de végétaux cultivés par des parasites phytopathogènes, caractérisé en ce que l'on applique un composé de formule I sur la plante ou son habitat.